# EUROPEAN PATENT APPLICATION

(11) **EP 2 979 687 A1**
(43) Date of publication of application: **03.02.2016**
(21) Application number: 14773441.2
(22) Date of filing: 26.03.2014
(51) Int. Cl.: A61K 8/87, A45D 29/18, A45D 31/00, A61K 8/81, A61Q 3/02, A61Q 3/04

(54) **ARTIFICIAL NAIL COMPOSITION, ARTIFICIAL NAIL, METHOD FOR FORMING ARTIFICIAL NAIL, AND NAIL ART KIT**

(30) Priority: 29.03.2013 JP 2013073038; 27.09.2013 JP 2013201915
(71) Applicant: FUJIFILM Corporation, Tokyo 106-8620 (JP)
(72) Inventor: ABE Junya, Haibara-gun Shizuoka 421-0396 (JP); OOHASHI Hidekazu, Haibara-gun Shizuoka 421-0396 (JP); USHIJIMA Kenta, Haibara-gun Shizuoka 421-0396 (JP)
(74) Representative: Hoffmann Eitle
(86) International application number: PCT/JP2014/058409
(87) International publication number: WO 2014/157272

(57) **Abstract**

To provide an artificial nail composition that has excellent stability over time and that can give an artificial nail having excellent surface gloss, removability, and adhesion, and to provide an artificial nail, a method for forming an artificial nail, and a nail art kit that employ the artificial nail composition.

An artificial nail composition comprises (Component A) an amino group-containing polymer.

## Description

The present invention relates to an artificial nail composition, an artificial nail, a method for forming an artificial nail, and a nail art kit.

In recent years, nail art, in which a design is applied to fingernails or toenails, has become popular, and techniques for attaching an artificial nail made of a synthetic resin (a false nail, a nail tip, etc.) to a nail (a real nail) have been developing. In recent years in particular, a nail decoration called an artificial nail, which is formed by coating a nail with a gel type curable composition for decoration comprising a urethane-based resin and a photopolymerizable monomer and then curing it by irradiation with UV, has been attracting attention due to reasons such as the finish being clear, adhesion to a nail being high and it being long lasting and, unlike an acrylic resin, there being no smell.

When this artificial nail is removed, conventionally the method mainly used is one in which a rough file (nail sander) is scraped against the artificial nail surface so that a removal liquid can easily penetrate, cotton impregnated with an organic solvent such as acetone (a removal liquid) is then placed on the artificial nail, this is wrapped with an aluminum foil, etc. and allowed to stand so that the artificial nail swells, and this swollen artificial nail is peeled off using a wooden spatula, etc.

Furthermore, as conventional artificial nail compositions, compositions described in Patent Documents 1 and 2 are known.
Patent Document 1: JP-A-2009-126833 (JP-A denotes a Japanese unexamined patent application publication)
Patent Document 2: JP-A-2-221214

The purpose of the present invention is to provide an artificial nail composition that has excellent stability over time and that can give an artificial nail having excellent surface gloss, removability, and adhesion, and to provide an artificial nail, a method for forming an artificial nail, and a nail art kit that employ the artificial nail composition.

The objects have been attained by means described in <1> or <14> to <16>. They are shown below together with <2> to <13>, which are preferred Embodiments.
<1> An artificial nail composition comprising (Component A) an amino group-containing polymer,
<2> the artificial nail composition according to <1>, wherein Component A is a polymer having an amino group in a side chain,
<3> the artificial nail composition according to <1> or <2>, wherein the amino group is a secondary amino group and/or a tertiary amino group,
<4> the artificial nail composition according to any one of <1> to <3>, wherein the amino group is a tertiary amino group,
<5> the artificial nail composition according to any one of <1> to <4>, wherein Component A is a polymer comprising a structure represented by Formula (I) below in a side chain, 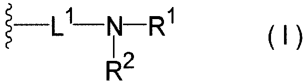 wherein in Formula (I) R¹ and R² independently denote a hydrogen atom or an alkyl group having 1 to 10 carbons, at least two selected from the group consisting of R¹, R², and L¹ may be bonded to each other to form a ring, L¹ denotes an alkylene group having 1 to 20 carbons, and the wavy line portion denotes the position of bonding to another structure,
<6> the artificial nail composition according to <5>, wherein R¹ and R² are independently alkyl groups having 1 to 4 carbons,
<7> the artificial nail composition according to any one of <1> to <6>, wherein Component A is a urethane-based polymer,
<8> the artificial nail composition according to <7>, wherein Component A is a urethane-based polymer having a polycarbonate structure or a polyester structure,
<9> the artificial nail composition according to <8>, wherein Component A is a urethane-based polymer having a polycarbonate structure,
<10> the artificial nail composition according to any one of <1> to <6>, wherein Component A is a polymer comprising a monomer unit represented by Formula (II) below, 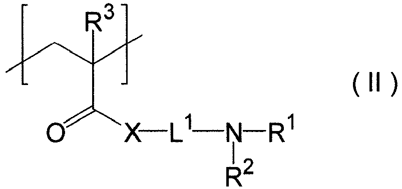 wherein in Formula (II) R¹ and R² independently denote a hydrogen atom or an alkyl group having 1 to 10 carbons, at least two selected from the group consisting of R¹, R², and L¹ may be bonded to each other to form a ring, L¹ denotes an alkylene group having 1 to 20 carbons, R³ denotes a hydrogen atom or an alkyl group having 1 to 3 carbons, and X denotes an oxygen atom or an NH group,
<11> the artificial nail composition according to <10>, wherein Component A is a copolymer comprising a monomer unit represented by Formula (II) above and a monomer unit derived from a compound selected from the group consisting of a styrene compound and a (meth)acrylate compound other than a monomer unit represented by Formula (II) above,
<12> the artificial nail composition according to any one of <1> to <9>, wherein Component A is a polymer comprising a monomer unit represented by Formula (III) below, 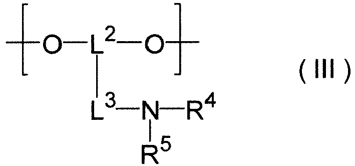 wherein in Formula (III) R⁴ and R⁵ independently denote a hydrogen atom or an alkyl group having 1 to 10 carbons, at least two selected from the group consisting of R⁴, R⁵, and L³ may be bonded to each other to form a ring, L² denotes a trivalent linking group, and L³ denotes an alkylene group having 1 to 20 carbons,
<13> the artificial nail composition according to any one of <1> to <12>, wherein the artificial nail composition further comprises (Component B) a polymerizable compound and (Component C) a photopolymerization initiator,
<14> an artificial nail comprising a layer formed by drying and/or exposing to light the artificial nail composition according to any one of <1> to <13>,
<15> a method for forming an artificial nail, comprising a step of forming a coated film by coating a human or animal nail or another artificial nail with the artificial nail composition according to any one of <1> to <13>, and a step of forming an artificial nail by drying and/or exposing to light the coated film,
<16> a nail art kit comprising the artificial nail composition according to any one of <1> to <13> and a removal liquid.

In accordance with the present invention, there can be provided an artificial nail composition having excellent stability over time and excellent surface gloss, removability, and adhesion of an artificial nail obtained thereby, an artificial nail employing the artificial nail composition, a method for forming an artificial nail, and a nail art kit.

The present invention is explained in detail below.

In the present specification, the notation 'xx (lower limit) to yy (upper limit)' means a numerical range that includes xx and yy. The same applies to 'yy (upper limit) to xx (lower limit)'. Furthermore, '(Component A) an amino group-containing polymer', etc. is also called simply 'Component A', etc.

In the present invention, 'mass%' has the same meaning as 'wt%', and 'parts by mass' has the same meaning as 'parts by weight'.

In the present invention, a combination of two or more preferred embodiments is a more preferred embodiment.

In the present specification, the representation of a group in a compound shown by a formula is as follows. When it is not clearly stated whether the group is substituted or unsubstituted, if the group can have a further substituent it includes not only the unsubstituted group but also a substituted group unless otherwise specified. For example, in the explanation of a formula, if it is stated that 'R denotes an alkyl group or an aryl group', this means that 'R denotes an unsubstituted alkyl group, a substituted alkyl group, an unsubstituted aryl group, or a substituted aryl group'. Furthermore, in the present specification, (meth)acrylic denotes a concept that includes both acrylic and methacrylic, and the same applies to (meth)acrylate, etc.

In the present specification, the representation of a polymer includes a copolymer.

### (Artificial nail composition)

The artificial nail composition of the present invention comprises (Component A) an amino group-containing polymer.

Furthermore, the artificial nail of the present invention is an artificial nail comprising a layer formed with the artificial nail composition of the present invention.

As a result of a detailed investigation by the present inventors, it has been found that due to it comprising Component A, the artificial nail composition of the present invention has excellent stability over time and can give an artificial nail having excellent surface gloss, removability, and adhesion.

Although the mechanism by which these effects are exhibited is not clear, part thereof is surmised to be as follows.

It is surmised that adhesion to a nail or adhesion to an upper layer that is optionally formed is exhibited by an unpaired electron of the amino group possessed by Component A. This effect of adhesion due to an unpaired electron is enhanced when there are a plurality of amino groups per molecule, and stress can be dispersed due to adsorption via multiple points. An artificial nail composition comprising an amino group-containing monomer such as one described in Patent Document 1 is subjected to radical polymerization or drying in air, and a low-molecular-weight component therefore remains after formation. It is surmised that in the present invention, adding a polymer in advance enables a stable molecular weight to be maintained, stable adsorption via multiple points to be achieved, and adhesion to be enhanced.

Furthermore, it is surmised that degradation of stability over time of the artificial nail composition when an amino group-containing component is used is due to the nitrogen atom of the amino group being oxidized and changed into an *N*-oxide. It is surmised that in the present invention, by adding Component A, that is, adding a polymer, the number of amino groups per molecule is increased, the influence of change to an N-oxide is suppressed, and the performance over time can be stabilized.

The artificial nail composition of the present invention is a composition that cures upon drying and/or exposure to light and forms an artificial nail above a human or animal nail or another artificial nail. Furthermore, an artificial nail formed using the artificial nail composition of the present invention can be removed by a conventional removal method using an organic solvent, but can also be easily removed with an acidic aqueous solution having a pH of no greater than 5.0.

The artificial nail in the present invention denotes a layer formed for the purpose of decoration and/or protection above a human or animal nail. Examples of said other artificial nail include a resin substrate having any shape for the purpose of decoration and/or protection of a nail.

A 'human and animal nail or another artificial nail' is also simply called a 'nail'.

The shape of the artificial nail is not particularly limited, and it may be formed into a desired shape. For example, it may be formed so as to cover a nail surface, may be formed only above part of a nail, or may be formed into a shape that is larger than a nail in order to extend the nail using a nail form, etc.

Furthermore, the thickness of the artificial nail composition of the present invention can be controlled by coating. The thickness is not particularly limited as long as it is in a range that is usually possessed by an artificial nail, but is preferably in the range of 20 to 1,500 µm from the viewpoint of practicality and removability.

The artificial nail composition of the present invention may be used suitably as any of a primer layer, a base layer, a color layer, and/or a top layer of an artificial nail.

Among them, from the viewpoint of removability it is preferable for a layer formed using the artificial nail composition of the present invention to be in contact with a nail.

Furthermore, an upper layer (face on the reverse side to a nail) or a lower layer (face between itself and a nail) of an artificial nail layer formed using the artificial nail composition of the present invention may comprise, separately, a primer layer, a base layer, a color layer, and/or a top layer for the purpose of imparting color, gloss, and adhesion.

The artificial nail composition of the present invention may be suitably used as a photocurable artificial nail composition (an 'artificial nail composition for a gel nail') and also as an artificial nail composition for a manicure liquid.

Furthermore, since the artificial nail composition of the present invention has excellent removability even when curing is carried out by exposure to light, it may particularly suitably be used as a photocurable artificial nail composition.

The photocurable artificial nail composition is an artificial nail composition that can be cured by actinic radiation. The 'actinic radiation' referred to here is actinic radiation that can give energy that generates an initiating species in the artificial nail composition by irradiation therewith, and includes UV, visible light, etc.

Various components that can be used in the artificial nail composition of the present invention are explained below.

### (Component A) Amino group-containing polymer

The artificial nail composition of the present invention comprises (Component A) an amino group-containing polymer.

The weight-average molecular weight of Component A is preferably at least 3,000, more preferably at least 5,000, and yet more preferably at least 5,000 but no greater than 250,000. When in this range, the artificial nail obtained has excellent adhesion and removability, and the stability over time is excellent.

The structure of Component A is not particularly limited and may be any structure, examples including a chain-form structure, a branched structure, a star structure, a crosslinked structure, and a net structure.

Component A may comprise an ethylenically unsaturated group. When Component A comprises an ethylenically unsaturated group, the ethylenically unsaturated group may be present on any of the main chain, a side chain, or a terminal of the polymer of Component A.

The amino group in Component A may be any of a primary amino group, a secondary amino group, and a tertiary amino group, but is preferably a secondary amino group or a tertiary amino group, and more preferably a tertiary amino group. With this embodiment, the artificial nail obtained has superior removability and water resistance, and synthesis is easy.

Furthermore, the amino group in Component A is preferably a monoalkylamino group or a dialkylamino group. With this embodiment, the artificial nail obtained has superior removability, adhesion, and water resistance.

Moreover, Component A may comprise an amino group on either the main chain or a side chain of the polymer, but from the viewpoint of synthesis it is preferably a polymer containing an amino group on a side chain.

Component A may have only one type of amino group or may have two or more types thereof.

Furthermore, the number of amino groups in Component A is not particularly limited; it may comprise any number, but it preferably comprises an amino group-containing monomer unit.

In addition, the 'monomer unit' referred to in the present invention includes not only a constituent unit derived strictly from a monomer but also a monomer unit modified by a polymer reaction involving a modification reaction, etc. carried out after obtaining the polymer.

Component A is preferably a polymer having a structure represented by Formula (I) below, and more preferably a polymer having a structure represented by Formula (I) below in a side chain. 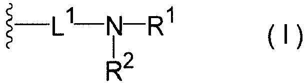 (In Formula (I), R¹ and R² independently denote a hydrogen atom or an alkyl group having 1 to 10 carbons, at least two selected from the group consisting of R¹, R², and L¹ may be bonded to each other to form a ring, L¹ denotes a divalent linking group, and the wavy line portion denotes the position of bonding to another structure.)

R¹ and R² in Formula (I) are independently preferably alkyl groups having 1 to 10 carbons from the viewpoint of achieving a balance between water resistance and solubility in an acid aqueous solution, and are particularly preferably methyl groups or ethyl groups.

Furthermore, in Formula (I), at least two selected from the group consisting of R¹, R², and L¹ may be bonded to each other to form a ring.

L¹ in Formula (I) may have a substituent having 1 to 20 carbons from the viewpoint of the film properties of the polymer, and is preferably an alkylene group in which some carbon atoms may be replaced by a heteroatom, more preferably an alkylene group having 1 to 20 carbons, an oxyalkylene group having 2 to 20 carbons, or a polyoxyalkylene group having 2 to 20 carbons, yet more preferably an alkylene group having 1 to 20 carbons or a polyoxyalkylene group having 2 to 20 carbons, particularly preferably an alkylene group having 1 to 20 carbons, and most preferably an alkylene group having 2 or 3 carbons.

Furthermore, the heteroatom is preferably a nitrogen atom or an oxygen atom, and more preferably an oxygen atom. When the heteroatom is a nitrogen atom, the remaining group on the nitrogen atom is preferably a hydrogen atom or an alkyl group, and more preferably an alkyl group.

Examples of a method for introducing a structure represented by Formula (I) above into a side chain of a polymer include a method in which a monomer having a structure represented by Formula (I) is polymerized by a known polymerization reaction such as polycondensation, polyaddition, addition condensation, or ionic polymerization (cationic polymerization/anionic polymerization).

Component A is preferably a polymer comprising a monomer unit represented by Formula (II) below from the viewpoint of film properties, ease of handling (viscosity), and introduction of an amino group synthetically. 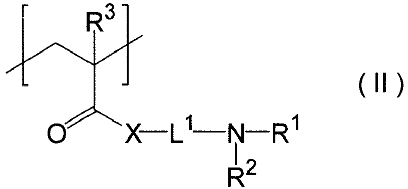 (In Formula (II), R¹ and R² independently denote a hydrogen atom or an alkyl group having 1 to 10 carbons, at least two selected from the group consisting of R¹, R², and L¹ may be bonded to each other to form a ring, L¹ denotes a divalent linking group, R³ denotes a hydrogen atom or an alkyl group having 1 to 3 carbons, and X denotes an oxygen atom or an NH group.)

R¹, R², and L¹ in Formula (II) have the same meanings as those of R¹, R², and L¹ in Formula (I) above, and preferred embodiments are also the same.

R³ in Formula (II) is preferably a hydrogen atom or a methyl group from the viewpoint of synthesis, and more preferably a methyl group.

X in Formula (II) is preferably an oxygen atom from the viewpoint of synthesis and water resistance of the artificial nail obtained.

When Component A comprises a monomer unit represented by Formula (II), it may comprise one type on its own or two or more types thereof, or may further comprise another monomer unit.

Examples of a monomer forming a monomer unit represented by Formula (II) include a compound represented by Formula (II-m) below. 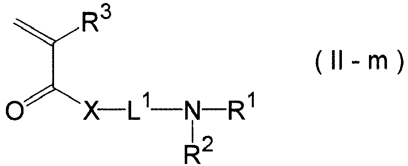 (In Formula (II-m), R¹ and R² independently denote a hydrogen atom or an alkyl group having 1 to 10 carbons, R¹ and R² or L¹ and R² may be bonded to each other to form a ring, L¹ denotes a divalent linking group, R³ denotes a hydrogen atom or an alkyl group having 1 to 3 carbons, and X denotes an oxygen atom or an NH group.)

R¹ to R³, L¹, and X in Formula (II-m) have the same meanings as those of R¹ to R³, L¹, and X in Formula (II) above, and preferred embodiments are also the same.

Furthermore, from the viewpoint of film properties, ease of handling (viscosity), and introduction of an amino group synthetically, Component A is preferably a polymer having a monomer unit represented by Formula (III) or Formula (III-b) below, and more preferably a urethane-based polymer having a monomer unit represented by Formula (III) below. 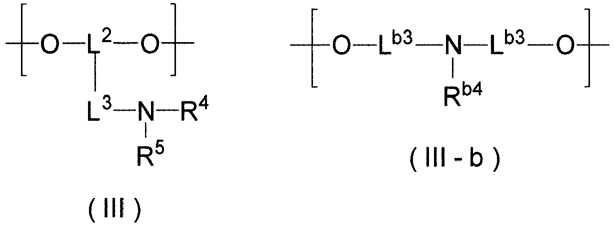 (In Formula (III) and Formula (III-b), R⁴, R⁵, and R^{b4} independently denote a hydrogen atom or an alkyl group having 1 to 10 carbons, at least two selected from the group consisting of R⁴, R⁵, and L³ may be bonded to each other to form a ring, R^{b4} and L^{b3} may be bonded to each other to form a ring, L² denotes a trivalent linking group, L³ denotes an alkylene group having 1 to 20 carbons, and the L^{b3}s independently denote an alkylene group having 1 to 20 carbons.)

From the viewpoint of achieving a balance between water resistance and solubility in an acid aqueous solution, R⁴ and R⁵ in Formula (III) are independently preferably alkyl groups having 1 to 10 carbons, and particularly preferably methyl groups or ethyl groups.

Furthermore, in Formula (III), at least two selected from the group consisting of R⁴, R⁵, and L³ may be bonded to each other to form a ring.

L² in Formula (III) is preferably a trivalent linking group having 2 to 20 carbons, more preferably a trivalent hydrocarbon group having 2 to 20 carbons, yet more preferably a trivalent aliphatic hydrocarbon group having 2 to 20 carbons, particularly preferably a trivalent aliphatic hydrocarbon group having 2 to 10 carbons, and most preferably a 1,1,2-ethanetriyl group that bonds to the two oxygen atoms in Formula (III) via the 1 and 2 positions.

Furthermore, from the viewpoint of the film properties of the polymer, L² in Formula (III) is preferably a trivalent aliphatic hydrocarbon group.

From the viewpoint of the film properties of the polymer, L³ in Formula (III) is preferably an alkylene group having 1 to 4 carbons, and particularly preferably a methylene group.

From the viewpoint of achieving a balance between water resistance and solubility in an acid aqueous solution, R^{b4} in Formula (III-b) is preferably an alkyl group having 1 to 10 carbons, more preferably an alkyl group having 2 to 8 carbons, yet more preferably an alkyl group having 4 to 8 carbons, and particularly preferably an n-butyl group.

From the viewpoint of the film properties of the polymer, the L^{b3}s in Formula (III-b) are independently preferably an alkylene group having 2 to 8 carbons, and particularly preferably an ethylene group.

Furthermore, from the viewpoint of synthesis, the two L^{b3}s are preferably the same group.

Moreover, in Formula (III-b), R^{b4} and L^{b3} may be bonded to each other to form a ring.

When Component A comprises a monomer unit represented by Formula (III) or Formula (III-b), it may comprise one type thereof or two or more types, and preferred examples of another monomer unit that can be present include a monomer unit derived from a diisocyanate component, which is described later.

Examples of a monomer that forms a monomer unit represented by Formula (III) include a compound represented by Formula (III-m) below.

Furthermore, examples of a monomer that forms a monomer unit represented by Formula (III-b) include a compound represented by Formula (III-bm) below. 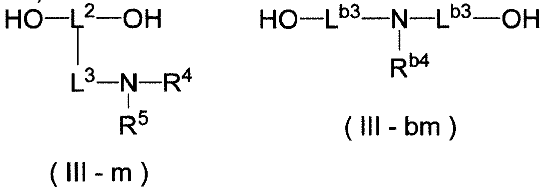 (In Formula (III-m) and Formula (III-bm), R⁴, R⁵, and R^{b4} independently denote a hydrogen atom or an alkyl group having 1 to 10 carbons, at least two selected from the group consisting of R⁴, R⁵, and L³ may be bonded to each other to form a ring, R^{b4} and L^{b3} may be bonded to each other to form a ring, L² denotes a trivalent linking group, L³ denotes an alkylene group having 1 to 20 carbons, and the L^{b3}s independently denote an alkylene group having 1 to 20 carbons.)

R⁴, R⁵, L², and L³ in Formula (III-m) have the same meanings as those of R⁴, R⁵, L², and L³ in Formula (III) above, and preferred embodiments are also the same.

R^{b4} and L^{b3} in Formula (III-bm) have the same meanings as those of R^{b4} and L^{b3} in Formula (III-b) above, and preferred embodiments are also the same.

Component A may be a polymer comprising only an amino group-containing monomer unit as a monomer unit, but when Component A comprises an amino group-containing monomer unit and another monomer unit, the content of the amino group-containing monomer unit is preferably 5 to 75 mole% relative to the total monomer units of Component A, more preferably 20 to 75 mole%, and yet more preferably 20 to 60 mole%. When in this range, the artificial nail obtained has superior removability and water resistance.

Furthermore, the amine value of Component A is preferably 0.001 to 8.5 mmol/g, more preferably 0.001 to 6.0 mmol/g, yet more preferably 0.005 to 4.0 mmol/g, and particularly preferably 0.01 to 2.0 mmol. When in this range, the removability and water resistance of the artificial nail obtained are superior.

As a method for measuring amine value, for example, it may be determined by weighing a sample in a beaker, adding acetic acid thereto, stirring so as to dissolve it, adjusting the measurement temperature to 25°C, and then carrying out titration by means of a titrator using a 0.1 N perchloric acid acetic acid solution as a titration reagent.

The amine value is the amount of perchloric acid consumed during titration and is expressed as the number of moles per g of a sample (solids content).

The polymer structure in Component A is not particularly limited and may be a desired polymer structure; examples include a polymer structure such as an acrylic polymer, a urethane-based polymer, a cellulose-based polymer, an ester-based polymer, an amide-based polymer, a vinyl-based polymer, an ether-based polymer, a styrene-based polymer, a carbonate-based polymer, a urea-based polymer, an ethylene-based polymer, and an amine-based polymer.

From the viewpoint of film properties, ease of handling (viscosity), and introduction of an amino group synthetically, Component A is preferably an ether-based polymer, an ethylene-based polymer (in particular a polyethyleneimine-based polymer), a urethane-based polymer, or an acrylic polymer, particularly preferably a urethane-based polymer or an acrylic polymer, and most preferably an acrylic polymer.

From the viewpoint of flexibility and removability of a cured film, a urethane-based polymer is most preferable.

With regard to the acrylic polymer, any polymer may be used suitably as long as it is a polymer obtained by polymerization of a known acrylic acid derivative (e.g. an acrylic acid ester such as methyl acrylate or ethyl acrylate, an acrylic acid amide such as acrylamide or acryloylmorpholine) or a methacrylic acid derivative (e.g. a methacrylic acid ester such as methyl methacrylate or ethyl methacrylate, a methacrylic acid amide such as methacrylamide or methacrylic acid isopropylamide). However, it should not be construed as being limited to these examples. Examples of a method for introducing an amino group into an acrylic polymer include a method involving polymerization or copolymerization of an amino group-containing (meth)acrylic acid derivative and a method involving introduction of an amino group by a polymer reaction. Examples of a method for introducing an ethylenically unsaturated group into an acrylic polymer include a method involving polymerization or copolymerization of an ethylenically unsaturated group-containing (meth)acrylic acid derivative and a method involving introduction of an ethylenically unsaturated group by a polymer reaction.

With regard to the urethane-based polymer, any polymer may be used suitably as long as it is a polyurethane formed from a known polyisocyanate compound (e.g. methylene diisocyanate, hexamethylene diisocyanate, trimethylhexamethylene diisocyanate, phenyl diisocyanate, tolylene diisocyanate, diphenylmethane diisocyanate, tolidine diisocyanate, naphthalene diisocyanate, triphenylmethane diisocyanate, tris(phenylisocyanete)thiophosphate, phenylene diisocyanate, lysine diisocyanate, xylylene diisocyanate, bis(isocyanatomethyl)cyclohexane, dicyclohexylmethane diisocyanate, isopropylidenebis(cyclohexylisocyanate), isophorone diisocyanate, dianisidine diisocyanate, or diphenylether diisocyanate) and a known polyol compound (e.g. an alkylenediol such as butanediol or hexanediol, an arylenediol such as p-hydroxystyrene, a polyether diol such as polyethylene glycol or polypropylene glycol, a polyester diol such as polyethylene glycol terephthalate, a polycarbonate diol such as polyethylene glycol carbonate, etc.). However, it should not be construed as being limited to these examples.

Examples of a method for introducing an amino group into a urethane-based polymer include a method involving polycondensation using a polyisocyanate compound and/or a polyol compound having a tertiary amino group or a protected amino group and a method involving introducing an amino group by a polymer reaction. A protected amino group may be deprotected by a known method after forming a polymer. The same applies below.

Examples of a method for introducing an ethylenically unsaturated group into a urethane-based polymer include a method involving introduction by a polymer reaction and a method involving polycondensation using an ethylenically unsaturated group-containing polyol compound or polyisocyanate compound. These compounds are not particularly limited, but with regard to an ethylenically unsaturated group-containing polyol, a (meth)acrylate-containing diol compound such as one described in JP-A-2010-100047 may suitably be used.

Among them, from the viewpoint of water resistance, it is preferably a urethane-based polymer comprising a polycarbonate structure, a polyester structure, a polybutadiene structure, or a hydrogenated polybutadiene structure, and more preferably a urethane-based polymer comprising a polycarbonate structure.

Preferred examples of a urethane-based polymer include a polycondensation product of a diisocyanate compound and a diol compound.

As the diol component forming the urethane-based polymer, it is preferable to use a polycarbonate diol, a polyester diol, a polybutadienediol, or a hydrogenated polybutadienediol from the viewpoint of water resistance, and it is more preferable to use a polycarbonate diol.

As the diisocyanate component forming same, it is preferable to use hexamethylene diisocyanate, tolylene diisocyanate, isophorone diisocyanate, diphenylmethane diisocyanate, or dicyclohexylmethane-4,4'-diisocyanate from the viewpoint of solubility of the artificial nail composition and mechanical strength of the artificial nail formed, and it is more preferable to use isophorone diisocyanate, methylenediphenyl diisocyanate, or dicyclohexylmethane-4,4'-diisocyanate. With regard to these compounds, one type may be used on its own or a plurality thereof may be used in combination.

Furthermore, the above desirable diol component/diisocyanate component may be used in combination with another diol component/diisocyanate component. Both water resistance and mechanical strength of an artificial nail can be enhanced by the use of a desirable diol component and a desirable diisocyanate component in combination.

With regard to the the cellulosic polymer, any cellulosic polymer such as carboxymethylcellulose, nitrocellulose, triacetylcellulose, etc. may be used suitably. However, it should not be construed as being limited to these examples. Examples of a method for introducing an amino group into a cellulose-based polymer include a method involving introducing an amino group by a polymer reaction. Examples of a method for introducing an ethylenically unsaturated group into a cellulose-based polymer include a method involving introducing an ethylenically unsaturated group by a polymer reaction.

With regard to the ester-based polymer, any polyester may be used suitably as long as it is a polyester formed from a known polycarboxylic acid compound (e.g. succinic acid, adipic acid, phthalic acid, etc.) and a known polyol compound. Furthermore, a polyester formed from a hydroxycarboxylic acid compound such as polylactic acid may also be suitably used. However, it should not be construed as being limited to these examples. Examples of a method for introducing an amino group into an ester-based polymer include a method in which polycondensation is carried out using a polycarboxylic acid compound, polyol compound and/or hydroxycarboxylic acid compound that have a tertiary amino group or a protected amino group and a method involving introducing an amino group by a polymer reaction. Examples of a method for introducing an ethylenically unsaturated group into an ester-based polymer include a method involving introducing an ethylenically unsaturated group by a polymer reaction.

With regard to the amide-based polymer, any polyamide may be used suitably as long as it is a polyamide formed from a known polycarboxylic acid compound and a known polyamine compound (e.g. ethylenediamine, phenylenediamine, etc.). Furthermore, a polyamino acid, which is a protein formed from amino acids, may also be used suitably. However, it should not be construed as being limited to these examples. Examples of a method for introducing an amino group into an amide-based polymer include a method in which polycondensation is carried out using a polycarboxylic acid compound having a tertiary amino group or a protected amino group and/or using a polyamine compound having a tertiary amino group or protected amino group that does not contribute to a polycondensation reaction, and a method involving introducing an amino group by a polymer reaction. Examples of a method for introducing an ethylenically unsaturated group into an amide-based polymer include a method involving introducing an ethylenically unsaturated group by a polymer reaction.

With regard to the vinyl-based polymer, any vinyl-based polymer may be used suitably as long as it is a vinyl-based polymer obtained by polymerization of a known vinyl compound (e.g. vinyl acetic acid, vinyl chloride, butadiene, etc.). However, it should not be construed as being limited to these examples. Examples of a method for introducing an amino group into a vinyl-based polymer include a method involving polymerization or copolymerization of an amino group-containing vinyl compound and a method involving introducing an amino group by a polymer reaction. Examples of a method for introducing an ethylenically unsaturated group into a vinyl-based polymer include a method involving introducing an ethylenically unsaturated group by a polymer reaction.

With regard to the ether-based polymer, any polyether may be used suitably as long as it is a polyether (e.g. polyethylene glycol, polypropylene glycol, etc.) formed from a known polyol compound. However, it should not be construed as being limited to these examples. Examples of a method for introducing an amino group into an ether-based polymer include a method in which polycondensation is carried out using a polyol compound having a tertiary amino group or a protected amino group and a method involving introducing an amino group by a polymer reaction. Examples of a method for introducing an ethylenically unsaturated group into an ether-based polymer include a method involving introducing an ethylenically unsaturated group by a polymer reaction.

With regard to the styrene-based polymer, any polystyrene may be used suitably as long as it is a polystyrene formed from a known styrene compound (e.g. styrene, 4-carboxystyrene, 4-acetoxystyrene, etc.). However, it should not be construed as being limited to these examples. Examples of a method for introducing an amino group into a styrene-based polymer include a method involving polymerization or copolymerization of a styrene derivative having an amino group and a method involving introducing an amino group by a polymer reaction. Examples of a method for introducing an ethylenically unsaturated group into a styrene-based polymer include a method involving introducing an ethylenically unsaturated group by a polymer reaction.

With regard to the carbonate-based polymer, any polycarbonate may be used suitably as long as it is a polycarbonate formed from a known carbonic acid derivative (e.g. phosgene, dimethyl carbonate, dimethyl carbonate, etc.) and a known polyol compound. However, it should not be construed as being limited to these examples. Examples of a method for introducing an amino group into a carbonate-based polymer include a method in which polycondensation is carried out using a polyol compound having a tertiary amino group or a protected amino group and a method involving introducing an amino group by a polymer reaction. Examples of a method for introducing an ethylenically unsaturated group into a carbonate-based polymer include a method involving introducing an ethylenically unsaturated group by a polymer reaction.

With regard to the urea-based polymer, any polyurea may be used suitably as long as it is a polyurea formed from a known polyisocyanate compound and a known polyamine compound. However, it should not be construed as being limited to these examples. Examples of a method for introducing an amino group into a urea-based polymer include a method in which polycondensation is carried out using a polyisocyanate compound having a tertiary amino group or a protected amino group and/or a polyamine compound having a tertiary amino group or protected amino group that does not contribute to a polycondensation reaction and a method involving introducing an amino group by a polymer reaction. Examples of a method for introducing an ethylenically unsaturated group into a urea-based polymer include a method involving introducing an ethylenically unsaturated group by a polymer reaction.

As an amine-based polymer, a known polyamine can be cited as an example. Examples of a method for introducing an ethylenically unsaturated group into an amine-based polymer include a method involving introducing an ethylenically unsaturated group by a polymer reaction.

Furthermore, Component A may be a copolymerized polymer such as a styrene-acrylic copolymer.

From the viewpoint of surface gloss, preferred examples of Component A include a homopolymer or copolymer of a monofunctional (meth)acrylate compound and a polycondensation product between a diisocyanate compound and a diol compound.

From the viewpoint of the water resistance of the artificial nail obtained, Component A is preferably a polymer comprising at least an amino group-containing monomer unit and a monomer unit that is derived from a water-insoluble monomer other than the amino group-containing monomer unit. Being water-insoluble is defined as meaning one that, when mixed with an equal volume of pure water under conditions of 1 atmosphere and 20°C, gives a mixed liquid that maintains a heterogeneous appearance.

Among the monomers that can be used, the water-insoluble monomer is preferably a styrene compound or a (meth)acrylate compound, more preferably a (meth)acrylate compound, and particularly preferably an alkyl (meth)acrylate ester.

That is, Component A preferably comprises, in addition to an amino group-containing monomer unit, a monomer unit derived from a compound selected from the group consisting of a styrene compound and a (meth)acrylate compound, more preferably a monomer unit derived from a (meth)acrylate compound, and yet more preferably a monomer unit derived from an alkyl (meth)acrylate ester.

In the production of Component A, preferred examples of the monomer that is copolymerizable with the amino group-containing monomer include a monofunctional (meth)acrylic acid ester such as methyl (meth)acrylate, ethyl (meth)acrylate, 2-ethylhexyl (meth)acrylate, 2-hydroxyethyl (meth)acrylate, or 2-hydroxypropyl (meth)acrylate, a monofunctional (meth)acrylamide such as 2-hydroxyethyl (meth)acrylamide, isopropylacrylamide, or morpholine acrylamide, a vinyl monomer such as *N*-vinylpyrrolidone or vinyl acetate, a polyfunctional (meth)acrylic acid ester such as ethylene glycol di(meth)acrylate, triethylene glycol di(meth)acrylate, tetraethylene glycol di(meth)acrylate, propylene glycol di(meth)acrylate, trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, 1,6-hexanediol di(meth)acrylate, a Cardo epoxy di(meth)acrylate, or neopentyl glycol di(meth)acrylate, and a urethane (meth)acrylate formed from a diisocyanate, a hydroxy group-containing (meth)acrylic acid derivative, and optionally a diol. It should not be construed as being limited to these examples.

From the viewpoint of the water resistance of the artificial nail obtained, Component A preferably further comprises, in addition to an amino group-containing monomer unit, a monomer unit represented by Formula (IV) below. 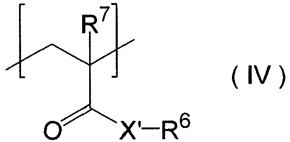 (In Formula (IV), R⁶ denotes a hydrogen atom, an alkyl group, or a group represented by Formula (V) below, R⁷ denotes a hydrogen atom or an alkyl group having 1 to 3 carbons, and X' denotes an oxygen atom or an NH group.) 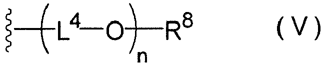 (In Formula (V), the L⁴s independently denote an ethylene group or a propylene group, n denotes an integer of 1 to 20, R⁸ denotes a hydrogen atom or an alkyl group, and the wavy line portion denotes the position of bonding to X'.)

From the viewpoint of the water resistance of the artificial nail obtained, R⁶ is preferably an alkyl group or a group represented by Formula (V) above, more preferably an alkyl group having 1 to 20 carbons or a group represented by Formula (V) above, yet more preferably an alkyl group having 3 to 10 carbons, and particularly preferably a butyl group.

From the viewpoint of synthesis, R⁷ is preferably a hydrogen atom or a methyl group, and more preferably a methyl group.

From the viewpoint of the water resistance of the artificial nail obtained and synthesis, X' is preferably an oxygen atom.

L⁴ is preferably an ethylene group.

From the viewpoint of the water resistance of the artificial nail obtained, n is preferably an integer of 1 to 10.

From the viewpoint of the water resistance of the artificial nail obtained, R⁸ is preferably an alkyl group having 1 to 10 carbons, and more preferably a methyl group.

Specific examples of Component A are shown below, but it should not be construed as being limited to these specific examples.

As Component A, polymers having the structures below are preferable, and polymers having the structures and weight-average molecular weights below are more preferable. The numerals on the bottom right of the parentheses of the monomer units denote molar ratio. The numerals on the bottom right of the parentheses of the alkyleneoxy chains denote the number of repeats. Furthermore, a constituent unit shown with a * means that the main chain terminal of the corresponding polymer has this structure bonded to the main chain via *.

**(Table 1)**

| | Structure | Mw |
|---|---|---|
| A-1 | 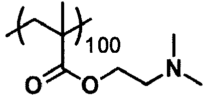 | 30,000 |
| A-2 | 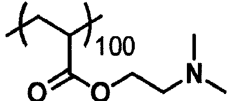 | 45,000 |
| A-3 | 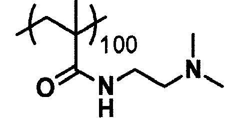 | 30,000 |
| A-4 | 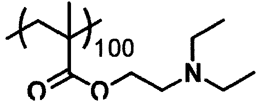 | 40,000 |
| A-5 | 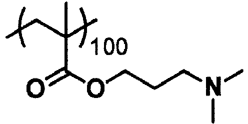 | 42,000 |
| A-6 | 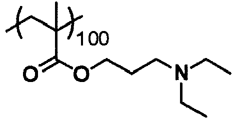 | 45,000 |
| A-7 | 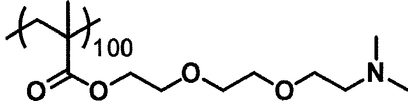 | 35,000 |
| A-8 | 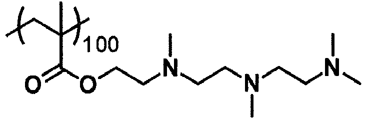 | 41,000 |
| A-9 | 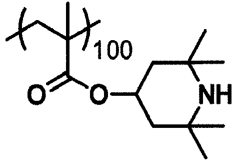 | 30,000 |
| A-10 | 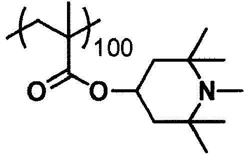 | 29,000 |

**(Table 2)**

| | Structure | Mw |
|---|---|---|
| A-11 | 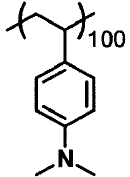 | 60,000 |
| A-12 | 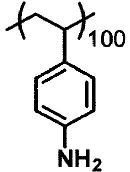 | 50,000 |
| A-13 | 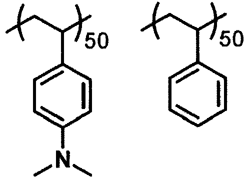 | 45,000 |
| A-14 | 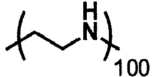 | 12,000 |
| A-15 | 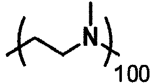 | 11,000 |
| A-16 | 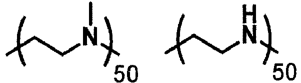 | 14,000 |
| A-17 | 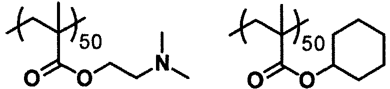 | 30,000 |
| A-18 | 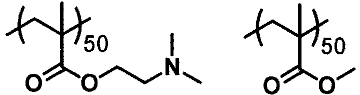 | 28,000 |
| A-19 | 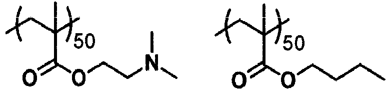 | 32,000 |
| A-20 | 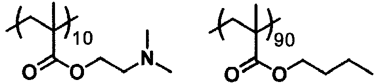 | 34,000 |

**(Table 3)**

| | Structure | Mw |
|---|---|---|
| A-21 | 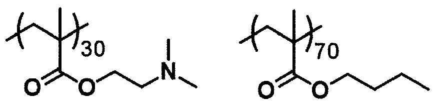 | 27,000 |
| A-22 | 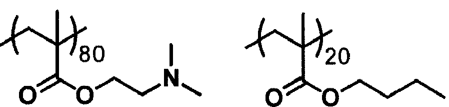 | 25,000 |
| A-23 | 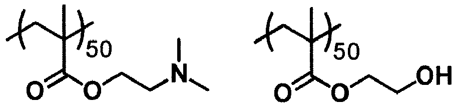 | 35,000 |
| A-24 | 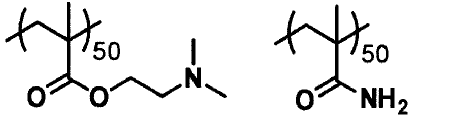 | 32,000 |
| A-25 | 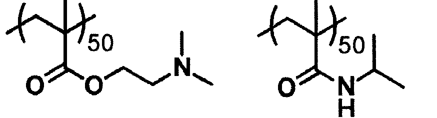 | 30,000 |
| A-26 | 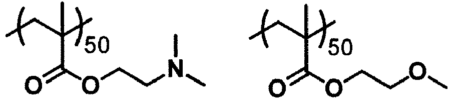 | 28,000 |
| A-27 | 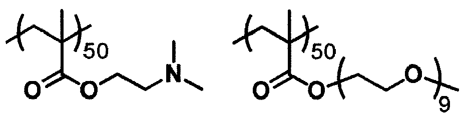 | 32,000 |
| A-28 | 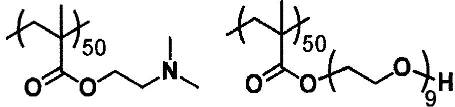 | 35,000 |
| A-29 | 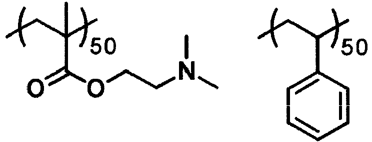 | 30,000 |
| A-30 | 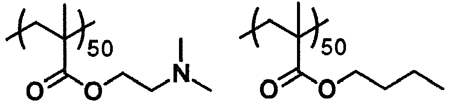 | 7,500 |

**(Table 4)**

| | Structure | Mw |
|---|---|---|
| A-31 | 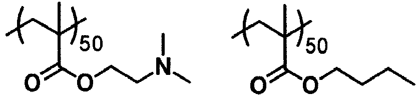 | 300,000 |
| A-32 | 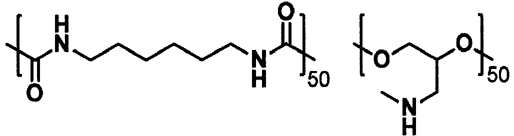 | 25,000 |
| A-33 | 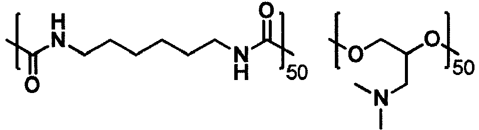 | 22,000 |
| A-34 | 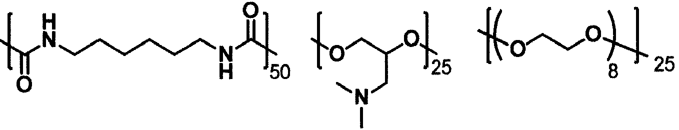 | 18,000 |
| A-35 | 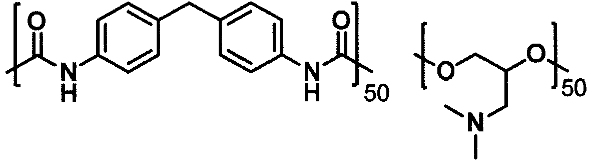 | 26,000 |
| A-36 | 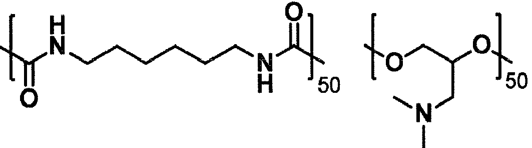 | 25,000 |
| A-37 | 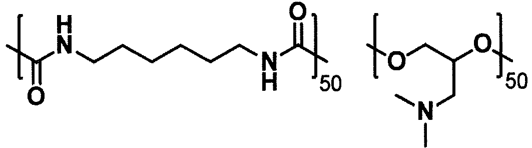 | 70,000 |

**(Table 5)**

| | Structure | Mw |
|---|---|---|
| A-38 | 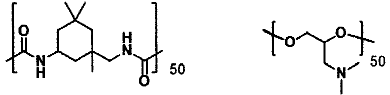 | 20,000 |
| A-39 | 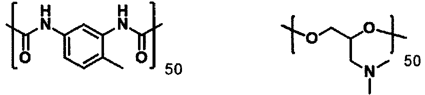 | 21,000 |
| A-40 | 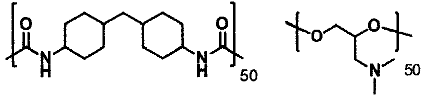 | 20,000 |
| A-41 | 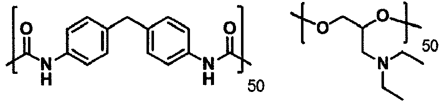 | 21,000 |
| A-42 | 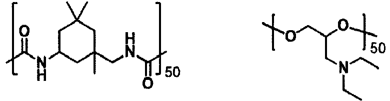 | 21,000 |
| A-43 | 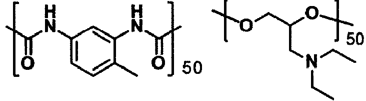 | 22,000 |
| A-44 | 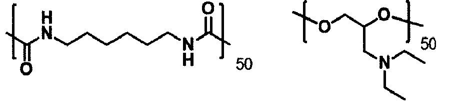 | 21,000 |
| A-45 | 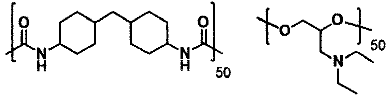 | 19,000 |
| A-46 | 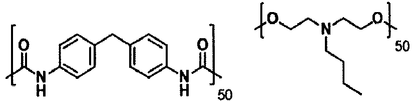 | 21,000 |
| A-47 | 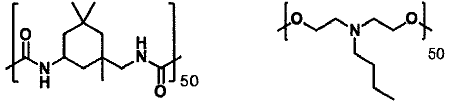 | 22,000 |

**(Table 6)**

| | Structure | Mw |
|---|---|---|
| A-48 | 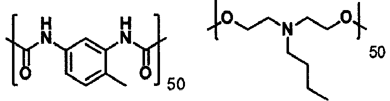 | 21,000 |
| A-49 | 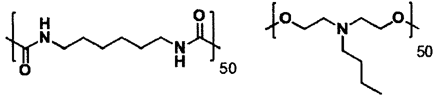 | 22,000 |
| A-50 | 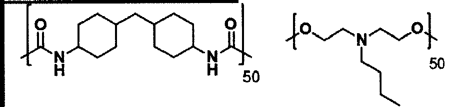 | 21,000 |
| A-51 | 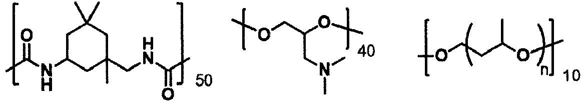 | 20,000 n=40 |
| A-52 | 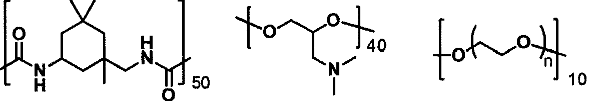 | 19,000 n=40 |
| A-53 | 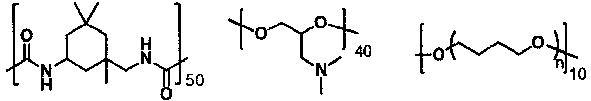 | 18,000 n=35 |
| A-54 | 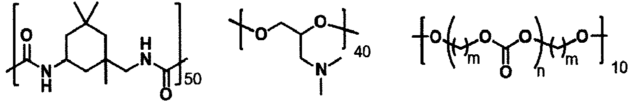 | 21,000 m=5 n=5 |
| A-55 | 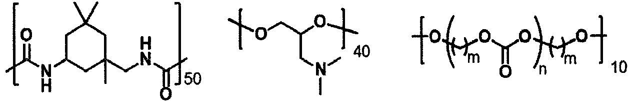 | 7,500 m=5 n=5 |
| A-56 | 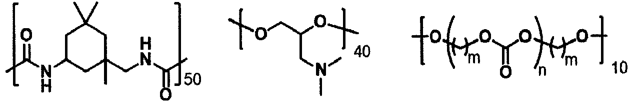 | 100,000 m=5 n=5 |
| A-57 | 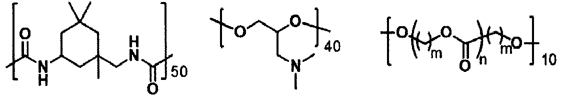 | 21,000 m=5 n=5 |

**(Table 7)**

| | Structure | Mw |
|---|---|---|
| A-58 | 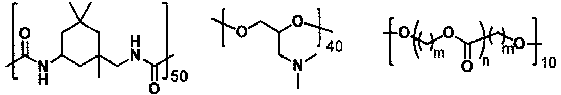 | 7,500 m=5 n=5 |
| A-59 | 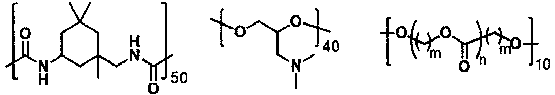 | 100,000 m=5 n=5 |
| A-60 | 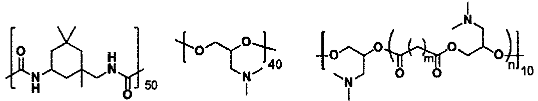 | 20,000 m=5 n=4 |
| A-61 | 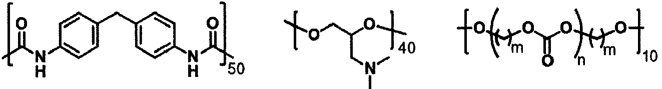 | 19,000 m=5 n=5 |
| A-62 | 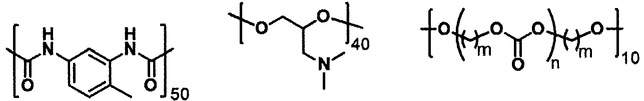 | 23,000 m=5 n=5 |
| A-63 | 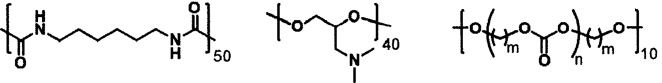 | 20,000 m=5 n=5 |
| A-64 | 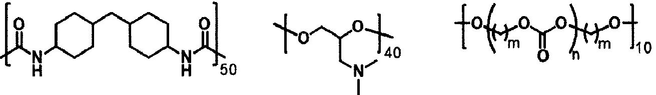 | 19,000 m=5 n=5 |
| A-65 | 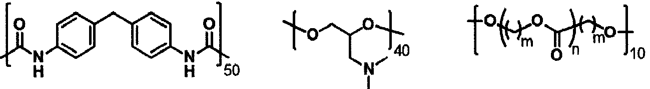 | 22,000 m=5 n=5 |
| A-66 | 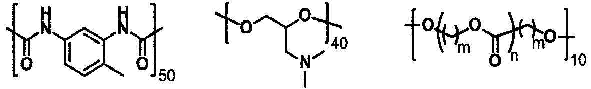 | 22,000 m=6 n=5 |
| A-67 | 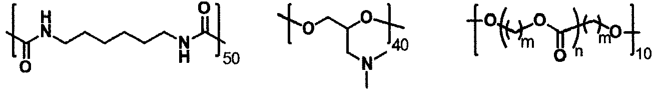 | 21,000 m=6 n=5 |

**(Table 8)**

| | Structure | Mw |
|---|---|---|
| A-68 | 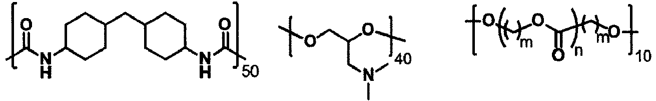 | 19,000 m=6 n=5 |
| A-69 | 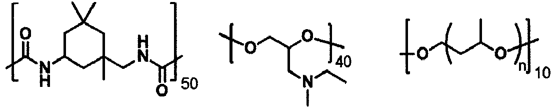 | 20,000 n=40 |
| A-70 | 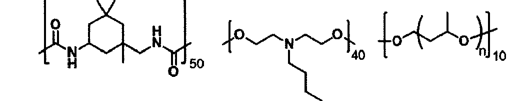 | 21,000 n=40 |
| A-71 | 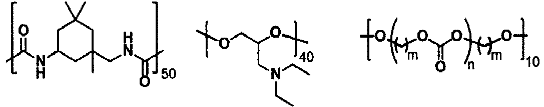 | 22,000 m=6 n=5 |
| A-72 | 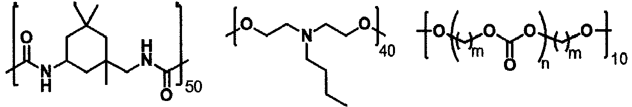 | 21,000 m=6 n=5 |
| A-73 | 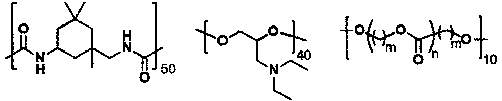 | 25,000 m=6 n=5 |
| A-74 | 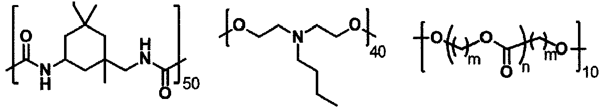 | 19,000 m=6 n=5 |
| A-75 | 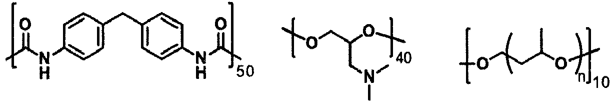 | 21,000 n=40 |
| A-76 | 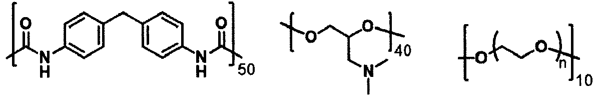 | 20,000 n=40 |
| A-77 | 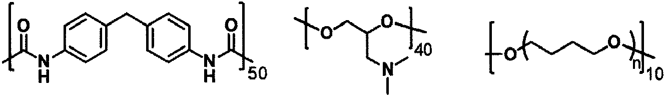 | 19,000 n=40 |

**(Table 9)**

| | Structure | Mw |
|---|---|---|
| A-78 | 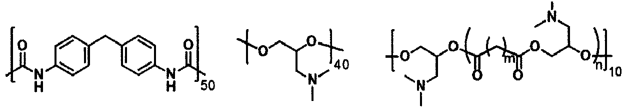 | 19,000 m=5 n=4 |
| A-79 | 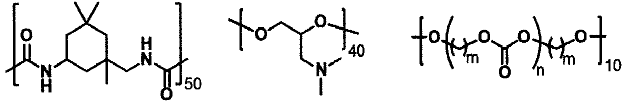 | 50,000 m=5 n=8 |
| A-80 | 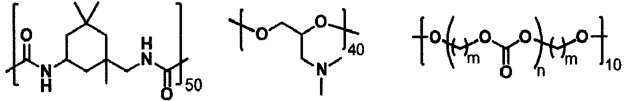 | 51,000 m=5 n=12 |
| A-81 | 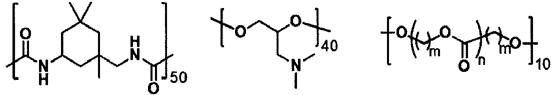 | 50,000 m=5 n=7 |
| A-82 | 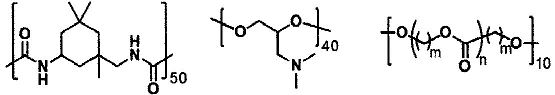 | 51,000 m=5 n=14 |
| A-83 | 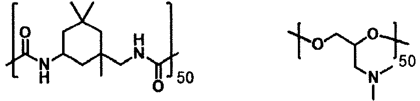 | 7,500 |
| A-84 | 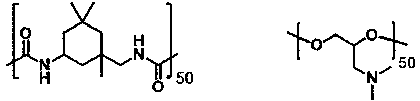 | 100,000 |
| A-85 | 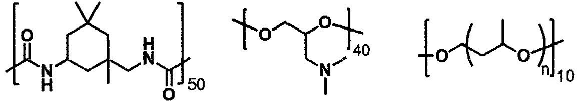 | 21,000 n=5 |
| A-86 | 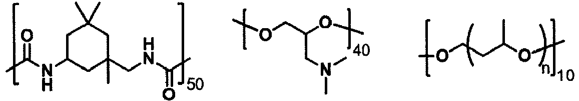 | 20,000 n=5 |
| A-87 | 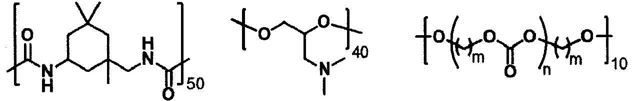 | 22,000 m=5 n=5 |
| A-88 | 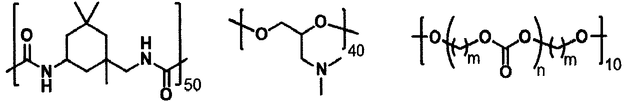 | 21,000 m=5 n=5 |

**(Table 10)**

| | Structure | Mw |
|---|---|---|
| A-89 | 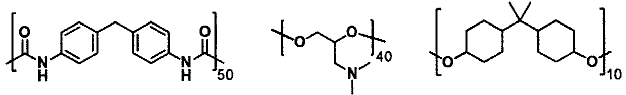 | 20,000 |
| A-90 | 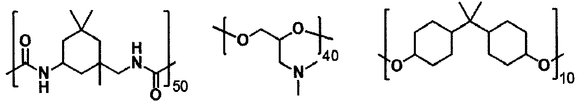 | 21,000 |
| A-91 | 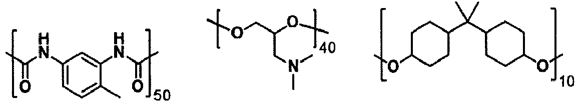 | 20,000 |
| A-92 | 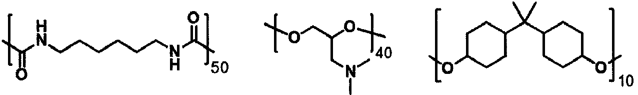 | 22,000 |
| A-93 | 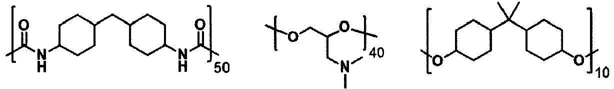 | 24,000 |
| A-94 | 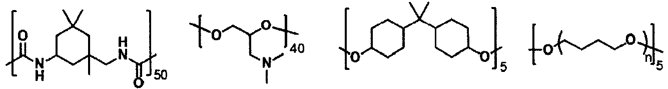 | 25,000 n=40 |

**(Table 11)**

| | Structure | Mw |
|---|---|---|
| A-95 | 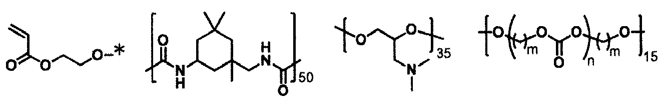 | 32,000 m=6,n=5 |
| A-96 | 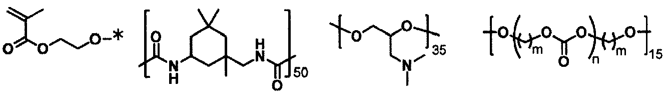 | 30,000 m=6,n=5 |
| A-97 | 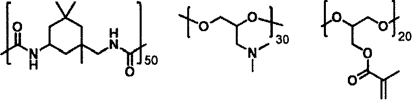 | 32,000 |
| A-98 | 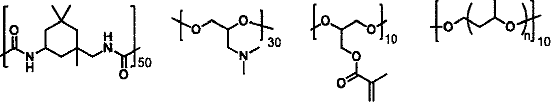 | 31,000 n=40 |
| A-99 | 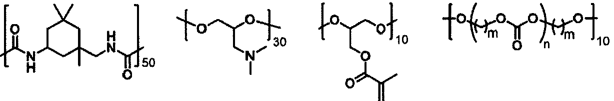 | 28,000 m=6,n=5 |
| A-100 | 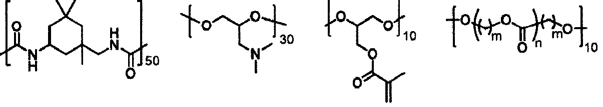 | 27,000 m=6,n=5 |
| A-101 | 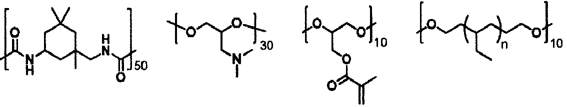 | 26,000 n=35 |
| A-102 | 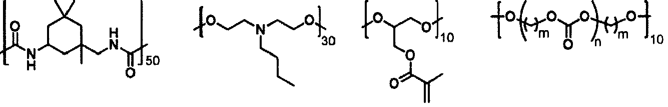 | 28,000 m=6,n=5 |
| A-103 | 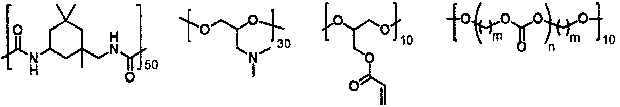 | 29,000 m=6,n=5 |
| A-104 | 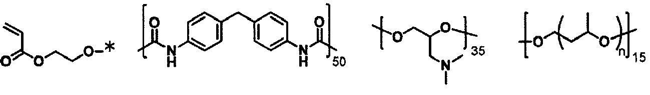 | 32,000 n=35 |

**(Table 12)**

| | Structure | Mw |
|---|---|---|
| A-105 | 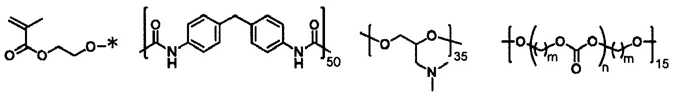 | 32,000 m=6, n=5 |
| A-106 | 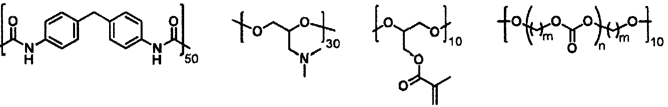 | 31,000 m=6,n=5 |
| A-107 | 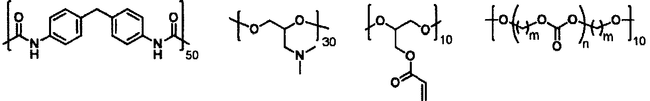 | 29,000 m=6,n=5 |
| A-108 | 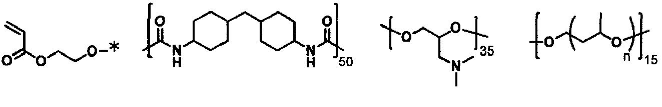 | 31,000 n=40 |
| A-109 | 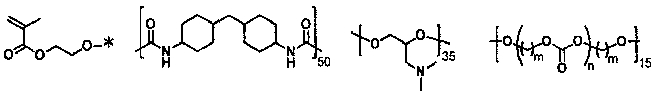 | 28,000 m=6,n=5 |
| A-110 | 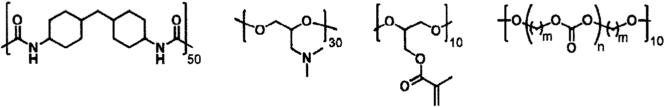 | 26,000 m=6,n=5 |
| A-111 | 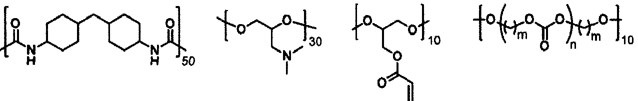 | 27,000 m=6,n=5 |
| A-112 | 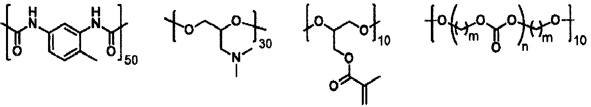 | 31,000 m=6,n=5 |
| A-113 | 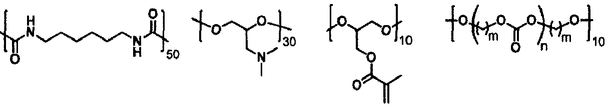 | 32,000 m=6,n=5 |
| A-114 | 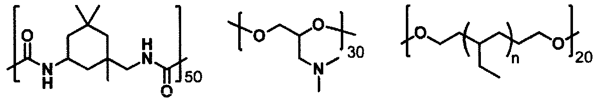 | 26,000 n=35 |

With regard to Component A, one type may be used on its own or two or more types may be used in combination.

The content of Component A in the artificial nail composition of the present invention is not particularly limited but is preferably 1 to 95 mass% relative to the total mass of the artificial nail composition, and more preferably 5 to 95 mass%. When in this range, the removability and adhesion of the artificial nail obtained are superior. When the solvent content in the artificial nail composition of the present invention is less than 10 mass%, the content of Component A is yet more preferably 5 to 90 mass% relative to the total mass of the artificial nail composition, and particularly preferably 10 to 90 mass%.

Furthermore, when the solvent content in the artificial nail composition of the present invention is 10 mass% or greater, the content of Component A is yet more preferably 30 to 80 mass% relative to the total mass of the artificial nail composition, and particularly preferably 40 to 75 mass%.

The artificial nail composition of the present invention may comprise a polymer other than Component A for the purpose of improving film properties or surface gloss.

From the viewpoint of the film properties, the polymer other than Component A is preferably an acrylic polymer or a urethane-based polymer. Furthermore, for the purpose of improving coating properties, a polymer known as a viscosity-adjusting agent (a thickener, etc.) may be added.

When the artificial nail composition of the present invention comprises a polymer other than Component A, the content of the polymer other than Component A is preferably less than the content of Component A, more preferably no greater than 40 mass% relative to the total mass of the artificial nail composition, yet more preferably no greater than 30 mass%, and still more preferably no greater than 20 mass%.

### (Component B) Polymerizable compound

The artificial nail composition of the present invention preferably comprises (Component B) a polymerizable compound. Due to it comprising Component B, the artificial nail composition of the present invention may be suitably used as a photocurable artificial nail composition.

When the artificial nail composition of the present invention comprises Component B, it preferably further comprises (Component C) a photopolymerization initiator, which is described below. Due to it comprising Component B and Component C, the artificial nail composition of the present invention may be suitably used as a photocurable artificial nail composition.

Component B may be either a radically polymerizable compound or a cationically polymerizable compound, but is preferably a radically polymerizable compound.

Component B is preferably an ethylenically unsaturated compound.

The radically polymerizable compound is a compound having a radically polymerizable ethylenically unsaturated bond and may be any compound as long as it comprises at least one radically polymerizable ethylenically unsaturated bond per molecule, and it includes one having a chemical form such as a monomer or an oligomer. With regard to the radically polymerizable compound, one type may be used on its own or two or more types may be used in combination at any ratio in order to improve desired properties. Component B is a compound other than Component A, is preferably a compound having a molecular weight of less than 10,000, and is more preferably a compound having a molecular weight of less than 5,000.

Examples of the radically polymerizable ethylenic compound include unsaturated carboxylic acids such as acrylic acid, methacrylic acid, itaconic acid, crotonic acid, isocrotonic acid, and maleic acid, and salts thereof, an ethylenically unsaturated group-containing anhydride, acrylonitrile, styrene and, furthermore, radically polymerizable compounds like oligomers such as various types of unsaturated polyesters, unsaturated polyethers, unsaturated polyamides, and unsaturated urethane.

Examples of other polymerizable compound include an acrylic derivatives such as 2-ethylhexyl(meth)acrylate, 2-hydroxyethyl(meth)acrylate, butoxyethyl(meth)acrylate, carbitol (meth)acrylate, cyclohexyl (meth)acrylate, benzyl (meth)acrylate, methyl (meth)acrylate, ethyl (meth)acrylate, *n*-propyl (meth) acrylate, isopropyl (meth)acrylate, *n*-butyl (meth)acrylate, allyl (meth)acrylate, glycidyl (meth)acrylate, *N*-methylol (meth)acrylamide, diacetone (meth)acrylamide, and epoxy (meth)acrylate, allyl compound derivatives such as allyl glycidyl ether, diallyl phthalate, triallyl trimellitate, etc., and more specifically, commercially available or industrially known radically polymerizable or crosslinking monomers and oligomers, such as those described in 'Kakyozai Handobukku' (Crosslinking Agent Handbook), Ed. S. Yamashita (Taiseisha, 1981); 'UV/EB Koka Handobukku (Genryo)' (UV/EB Curing Handbook (Starting Materials)) Ed. K. Kato (Kobunshi Kankoukai, 1985); 'UV/EB Koka Gijutsu no Oyo to Shijyo' (Application and Market of UV/EB Curing Technology), p. 79, Ed. RadTech (CMC, 1989); and E. Takiyama 'Poriesuteru Jushi Handobukku' (Polyester Resin Handbook), (The Nikkan Kogyo Shimbun Ltd., 1988) may be used.

Monomers used by synthesis of Component A may be used as Component B.

From the viewpoint of removability, adhesion and gloss of an artificial nail that is obtained, preferred examples of Component B include an ethylenically unsaturated compound having hydroxy group or an ethylenically unsaturated compound having (poly)alkyleneoxy group, more preferably a (meth)acrylate compounds having hydroxy group or a (meth)acrylate compounds having (poly)alkyleneoxy group, yet more preferably a (meth)acrylate compounds having hydroxy group and especially preferably a monofunctional (meth)acrylate compounds having hydroxy group.

Preferred examples of a (meth)acrylate compounds having hydroxy group include hydroxyalkyl(meth)acrylate such as 2-hydoxyethyl(meth)acrylate, 2-hydroxypropyl(meth)acrylate, 3-hydroxypropyl(meth)acrylate, 2,3-dihydroxypropyl(meth)acrylate and 4-hydroxybutyl(meth)acrylate, polyethyleneglycolmono(meth)acrylate, polypropyleneglycolmono(meth)acrylate, poly(ethyleneglycol/propyleneglycol)-mono(meth)acrylate, polyethyleneglycol/polypropyleneglycol-mono(meth)acrylate, poly(ethyleneglycol/tetramethyleneglycol)-mono(meth)acrylate, poly(propyleneglycol/tetramethyleneglycol)-mono(meth)acrylate and polypropyleneglycol/polybuthyleneglycol-mono(meth)acrylate.

Preferred examples of the (poly)alkyleneoxy group-containing (meth)acrylate compound include methoxypolyethylene glycol-(meth)acrylate, octoxypolyethylene glycol-polypropylene glycol-(meth)acrylate, lauroxypolyethylene glycol-(meth)acrylate, stearoxypolyethylene glycol-(meth)acrylate, phenoxypolyethylene glycol-(meth)acrylate, phenoxypolyethylene glycol-polypropylene glycol-(meth)acrylate, nonylphenoxy-polyethylene glycol-(meth)acrylate, nonylphenoxy-polypropylene glycol-(meth)acrylate, and nonylphenoxy-poly(ethylene glycol-propylene glycol)-(meth)acrylate.

As the hydroxy group-containing (meth)acrylate compound and the (poly)alkyleneoxy group-containing (meth)acrylate compound, commercial products may be used. Representative examples include Blemmer E, Blemmer PE-90, Blemmer PE-200, Blemmer PE-350, Blemmer P, Blemmer PP-1000, Blemmer PP-500, Blemmer PP-800, Blemmer 50PEP-300, Blemmer 70PEP-350B, Blemmer 55PET-800, the Blemmer PPT series, Blemmer 10PPB-500B, Blemmer AE-90, Blemmer AE-200, Blemmer AE-400, Blemmer AP-150, Blemmer AP-400, Blemmer AP-550, Blemmer PME-100, Blemmer PME-200, Blemmer PME-400, Blemmer PME-1000, Blemmer 50POEP-800B, Blemmer PLE-200, Blemmer PSE-400, Blemmer PSE-1300, Blemmer PAE-50, Blemmer PAE-100, Blemmer 43PAPE-600B, Blemmer AME-400, the Blemmer ALE series, Blemmer ANP-300, Blemmer 75ANP-600, Blemmer AAE-50, and Blemmer AAE-300 (all manufactured by NOF Corporation).

When a hydroxy group-containing ethylenically unsaturated compound is used, the number of hydroxy groups in the compound is preferably 1 to 10, more preferably 1 to 5, yet more preferably 1 to 3, and particularly preferably 1.

When a (poly)alkyleneoxy group-containing ethylenically unsaturated compound is used, the number of repeating units of the alkyleneoxy group in the compound is preferably 1 to 25, more preferably 1 to 15, and yet more preferably 1 to 10.

Among them, Component B preferably comprises a hydroxyalkyl (meth)acrylate compound, more preferably comprises 2-hydroxyethyl (meth)acrylate, 2-hydroxypropyl (meth)acrylate, or 3-hydroxypropyl (meth)acrylate, and yet more preferably comprises 2-hydroxyethyl (meth)acrylate.

From the viewpoint of the water resistance of the artificial nail obtained, Component B preferably comprises an ethylenically unsaturated compound containing an alkyl group having 3 or more carbons, more preferably an ethylenically unsaturated compound containing an alkyl group having 5 or more carbons, and yet more preferably an ethylenically unsaturated compound containing an alkyl group having 8 or more carbons. These alkyl groups may have a branched and/or cyclic structure.

Examples of the alkyl group-containing ethylenically unsaturated compound include, but are not limited to, propyl (meth)acrylate, butyl (meth)acrylate, hexyl (meth)acrylate, cyclohexyl (meth)acrylate, 2-ethylhexyl (meth)acrylate, lauryl methacrylate, isodecyl (meth)acrylate, tridecyl (meth)acrylate, stearyl (meth)acrylate, or isomers thereof.

From the viewpoint of the solubility of a component other than Component B, Component B preferably comprises a (meth)acrylamide compound. Examples of the (meth)acrylamide compound include, but are not limited to, *N*-hydroxyethyl (meth)acrylamide, *N*-butoxymethyl (meth)acrylamide, *N*-*t*-butyl (meth)acrylamide, *N,N*-dimethyl (meth)acrylamide, *N,N*-diethyl (meth)acrylamide, *N*-dodecyl (meth)acrylamide, *N*-hydroxymethyl (meth)acrylamide, *N*-isopropyl (meth)acrylamide, *N*-phenyl (meth)acrylamide, *N*-isobutoxymethyl (meth)acrylamide, and *N*-methoxymethyl acrylamide.

From the viewpoint of the heat resistance of the artificial nail composition obtained, Component B preferably comprises an ethylenically unsaturated compound having a Tg (glass transition temperature) as a homopolymer of at least 50°C, more preferably comprises an ethylenically unsaturated compound having a Tg of at least 80°C, and yet more preferably comprises an ethylenically unsaturated compound having a Tg of at least 100°C.

Examples of the ethylenically unsaturated compound having a Tg as a homopolymer of at least 50°C include, but are not limited to, methyl (meth)acrylate, *t*-butyl (meth)acrylate, benzyl methacrylate, isobornyl (meth)acrylate, and adamantyl (meth)acrylate.

From the viewpoint of the removability and film properties of the artificial nail obtained, Component B preferably comprises a urethane bond-containing polymerizable compound, more preferably comprises a urethane bond-containing polyfunctional polymerizable compound, and yet more preferably comprises a urethane bond-containing polyfunctional (meth)acrylate compound.

Component B preferably comprises a polyfunctional polymerizable compound having a molecular weight (weight-average molecular weight) of at least 1,000, and more preferably comprises a polyfunctional polymerizable compound having a molecular weight (weight-average molecular weight) of at least 3,000. Due to it comprising a polyfunctional polymerizable compound having a molecular weight of at least 1,000, it is possible to control the crosslink density and prevent a film from becoming brittle, thus improving the durability of the artificial nail formed.

Preferred examples of the urethane bond-containing polyfunctional (meth)acrylate compound include a urethane-based addition polymerizable compound produced using an addition reaction between an isocyanate group and a hydroxy group, and specific examples thereof include a vinylurethane compound containing two or more polymerizable vinyl groups per molecule formed by adding a hydroxy group-containing vinyl monomer represented by Formula (A) below to an isocyanate compound containing two or more isocyanate groups per molecule described in JP-B-48-41708 (JP-B denotes a Japanese examined patent application publication).

CH₂=C(R⁷)COOCH₂CH(R⁸)OH (A)

(Here, R⁷ and R⁸ independently denote a hydrogen atom or a methyl group.)

Among them, the isocyanate compound is preferably hexamethylene diisocyanate, 2,2,4-trimethylhexamethylene diisocyanate, 2,4,4-trimethylhexamethylene diisocyanate, or 2,4-dimethyl diisocyanate, more preferably 2,2,4-trimethylhexamethylene diisocyanate, 2,4,4-trimethylhexamethylene diisocyanate, or 2,4-dimethyl diisocyanate, and yet more preferably 2,4-dimethyl diisocyanate.

Furthermore, as the isocyanate compound, for the purpose of controlling molecular weight, a polyester-based diisocyanate, a polyether-based diisocyanate, or a polycarbonate-based diisocyanate may suitably be used. These diisocyanate compounds may be obtained by reacting a polyether diol, a polyester diol, or a polycarbonate diol with a diisocyanate compound described above or another compound such as isophorone diisocyanate or methylenediphenyl diisocyanate. Here, from the viewpoint of the film properties or water resistance of an artificial nail, a urethane-based addition polymerizable compound having a polyester structure or a polycarbonate structure (more preferably a diol compound and/or a diisocyanate compound) may preferably be used.

With regard to Component B, one type may be used on its own or two or more types thereof may be used in combination.

The content of Component B in the artificial nail composition of the present invention is not particularly limited, but is preferably 1 to 95 mass% relative to the total mass of the artificial nail composition, more preferably 1 to 90 mass%, yet more preferably 5 to 90 mass%, and particularly preferably 5 to 85 mass%. When in this range, the removability and water resistance of the artificial nail obtained are superior.

### (Component C) Photopolymerization initiator

The artificial nail composition of the present invention preferably comprises (Component C) a photopolymerization initiator. Due to it comprising Component C, the artificial nail composition of the present invention may suitably be used as a photocurable artificial nail composition.

Examples of the photopolymerization initiator include a radical photopolymerization initiator and a cationic photopolymerization initiator, but a radical photopolymerization initiator is preferable.

As the photopolymerization initiator, a known photopolymerization initiator may be used. With regard to the photopolymerization initiator that can be used in the present invention, one type may be used on its own or two or more types may be used in combination. Furthermore, a radical photopolymerization initiator and a cationic photopolymerization initiator may be used in combination.

The photopolymerization initiator that can be used in the present invention is a compound that absorbs light and forms a polymerization-initiating species. Examples of light include γ-rays, β-rays, an electron beam, UV, visible light, and infrared, and UV and/or visible light are preferable.

As the photopolymerization initiator that can be used in the present invention, those below can be cited as preferred examples. But the present invention should not be construed as being limited thereto.

An acetophenone compound (for example, 1-hydroxycyclohexylphenylketone, acetophenone, 2,2-dimetoxy-2-phenylacetophenone, 2,2-dietoxyacetophenone, 2-hydroxy-2-methylpropiophenone, 4'-isopropyl-2-hydroxy-2-metylpropiophenone, 2-metyl-1-[4-(methylthio)phenyl]-2-morphorino-1-propane, 2-benzyl-2-dimethylamino-1-(4-morphorinophenyl)-butane-1-one, benzoin, benzoinmethylether, benzoinethylether or benzoinpropyl ether etc.);
a benzophenone compound (for example, benzophenone, 4.4'-bis(dimethylamino)benzophenone or 3,3-dimethyl-4-metoxy-benzophenone etc.);
a anthraquinone compound (for example, anthraquinone, 2-methylantraquinone, 2-ethylanthraquinone or *tert*-butylanthraquinone etc.);
a thioxanthone compound (for example, 2-chrolothioxantone, diethylthioxanthone, isopropylthioxanthone or diisopropylthioxanthone etc.);
a trihaloalkyl compound (for example, 2,4,6-(trichrolomethyl)triazine, 2,4-trichrolomethyl-6-(4-metoxyphenyl)triazine or tribromomethylphenylsulfone etc.);
a lophin dimer compound (for example, 2-(*o*-chrolophenyl)-4,5-diphenylimidazoledimer etc.);
an acridine compound (for example, 9-phenylacridine, 1,7-bis(9-acridinyl)heptane, 1,5-bis(9-acridinyl)pentane or 1,3-bis(9-acridinyl)propane etc.);
a phosphineoxide compound (for example, trimethylbenzoyldiphenylphosphineoxide, bis(2,4,6-trimethylbenzoyl)phenylphosphineoxide etc.);
a metallocene compound (for example, biscyclopentadienyl-bis(difluor-pyrryl-phenyl)titanium etc.);
an onium salt (for example, bis(4-*t*-butylphenyl)iodoniumtosylate or triphenylsulfoniumtosylate etc.);
an oxime ester compound (for example, 1-(4-phenylthiophenyl)-1,2-octandion-2-(O-benzoyloxime), 1-[9-ethyl-6-(2-methylbenzoyl)-9H-carbazol-3-yl]-ethanone-1-(*O*-acetyloxime) etc.).

Among them, preferred examples of Component C in the present invention include the photopolymerization initiator selected from the group consisting of an acetophenone compound, a phosphineoxide compound, a metallocene compound, and a lophin dimer compound, more preferably the photopolymerization initiator selected from the group consisting of an acetophenone compound and a phosphineoxide compound.

The content of component C in the artificial nail composition of the present invention is preferably 0.01 to 20 mass % relative to the total mass of artificial nail composition, more preferably 0.1 to 15 mass % and yet more preferably 0.5 to 12 mass %. When in this range, the curability of artificial nail composition is better, and adhesion, gloss and water resistance of an artificial nail that is obtained are excellent.

### (Component D) Solvent

From the viewpoint of coating properties, the artificial nail composition of the present invention may comprise (Component D) a solvent, but from the viewpoint of safety and ease of handling, the solvent content is preferably less than 10 mass% relative to the total mass of the artificial nail composition, and more preferably less than 5 mass%; it is particularly preferable that the artificial nail composition of the present invention does not comprise a solvent.

Any solvent may be used suitably as long as it is a known organic solvent for the solvent. Examples include an alcohol-based solvent such as ethanol, isopropanol, ethylene glycol monomethyl ether, ethylene glycol monobutyl ether, propylene glycol monomethyl ether, diethylene glycol monoethyl ether, or dipropylene glycol monomethyl ether and solvents based on acetates thereof, an ester-based solvent such as ethyl acetate or butyl acetate, a ketone-based solvent such as acetone, methyl ethyl ketone, methyl isobutyl ketone, or cyclohexanone, and an ether-based solvent such as tetrahydrofuran or methyl *t*-butyl ether. However, it should not be construed as being limited to these examples.

From the viewpoint of drying rate, the boiling point of the solvent is preferably 30°C to 130°C, more preferably 35°C to 100°C, yet more preferably 40°C to 90°C and particularly preferably 50°C to 85°C. When in this range, the drying property and coating property are excellent.

With regard to Component D, one type may be used on its own or two or more types thereof may be used in combination.

Component D preferably comprises an alcohol solvent and/or a ketone solvent, and more preferably an alcohol solvent and a ketone solvent.

When the artificial nail composition of the present invention comprises Component D, from the viewpoint of the coating properties and removability and adhesion of the artificial nail obtained, the content of Component D is preferably 1 to 90 mass% relative to the total mass of the artificial nail composition, more preferably 5 to 80 mass%, yet more preferably 10 to 80 mass%, and particularly preferably 15 to 50 mass%.

### <Other components>

The artificial nail composition of the present invention may optionally comprise, as an optional component, other than Component A to Component D, an additive component that can usually be added to an artificial nail composition within a range that does not impair the effects of the present invention. Examples of such an additive component include an antifoaming agent, a buffer agent, a chelating agent, a dispersant, a dye, a filler, a pigment, a preservative, a resin powder (e.g. poly(meth)acrylic acid, etc.), an inorganic powder (e.g. silica gel, etc.), a thickening agent, and a wetting agent. However, it should not be construed as being limited to these examples.

Furthermore, the amount of each component in the artificial nail composition of the present invention is not particularly limited, but it is preferable that, as a mass ratio in the artificial nail composition, Component A/Component B/Component C/Component D/Othe components = 10 to 90/5 to 85/0.5 to 12/0 to 50/0 to 40. When in this range, the coating properties are superior, and the removability and gloss of an artificial nail obtained are superior.

### (Artificial nail)

The artificial nail of the present invention is an artificial nail comprising a layer formed with the artificial nail composition of the present invention, and is preferably an artificial nail comprising a layer formed by drying and/or exposing to light the artificial nail composition of the present invention.

The artificial nail of the present invention is suitable as a nail polish such as a manicure or a pedicure, or as a gel nail.

With regard to the artificial nail of the present invention, at least part thereof may be formed with the artificial nail composition of the present invention and it may have another layer or structure, or the entirety of the artificial nail may be formed with the artificial nail composition of the present invention.

A layer formed with the artificial nail composition of the present invention may be used suitably for any of a primer layer, a base layer, a color layer, and/or a top layer in the artificial nail of the present invention.

Furthermore, the artificial nail of the present invention may have only one layer of the layer formed with the artificial nail composition of the present invention, or it may have two or more layers thereof.

Among them, from the viewpoint of removability it is preferable for at least one layer formed with the artificial nail composition of the present invention to be in direct contact with a human nail, etc.

Moreover, the thickness of the layer formed with the artificial nail composition of the present invention in the artificial nail of the present invention is not particularly limited but is preferably 10 to 2,000 µm, more preferably 20 to 1,500 µm, and yet more preferably 20 to 1,000 µm.

### (Method for forming artificial nail)

The method for forming an artificial nail of the present invention is not particularly limited as long as it is a method for forming an artificial nail using the artificial nail composition of the present invention, and is preferably a method comprising a step of forming a coated film by coating a human or animal nail or another artificial nail with the artificial nail composition of the present invention, and a step of forming an artificial nail by drying and/or exposing to light the coated film.

When the artificial nail composition of the present invention is formed into an artificial nail, a formation method involving either drying or exposure to light may desirably be selected.

The artificial nail composition of the present invention preferably comprises at least (Component B) a polymerizable compound and (Component C) a photopolymerization initiator, or comprises at least (Component D) a solvent.

Moreover, the thickness of the layer formed with the artificial nail composition of the present invention in the method for forming an artificial nail of the present invention is not particularly limited but is preferably 10 to 2,000 µm, more preferably 20 to 1,500 µm, and yet more preferably 20 to 1,000 µm.

### <Coating step>

The method for forming an artificial nail of the present invention preferably comprises a step (coating step) of forming a coated film by coating a human nail, etc. with the artificial nail composition of the present invention.

The other artificial nail is not particularly limited as long as it is a substrate used for an artificial nail, and examples include a resin substrate, an artificial nail formed by a method other than the method for forming an artificial nail of the present invention, and the artificial nail obtained by the method for forming an artificial nail of the present invention.

### <Drying or exposure step>

The method for forming an artificial nail of the present invention preferably comprises a step of forming an artificial nail by drying and/or exposing to light the coated film (drying or exposure step).

### -Drying step-

When forming the artificial nail by drying, the artificial nail composition of the present invention preferably comprises (Component D) a solvent.

A drying method is not particularly limited, and a known method may be employed.

Specific preferred examples include a method in which drying is carried out by allowing it to stand at room temperature (e.g. 10°C to 30°C), a method in which drying is carried out by allowing it to stand under a flow of gas, a method in which drying is carried out by heating, and a method in which the above methods are combined. A heating method is not particularly limited, and examples include a method in which it is allowed to stand under an atmosphere with a temperature that is higher than room temperature, a method in which it is allowed to stand under a flow of heated gas, and a method in which heating is carried out by means such as a heater or an infrared lamp (an IR lamp).

The drying time is not particularly limited and may be adjusted as appropriate according to the formulation of the artificial nail composition, the drying method, or the coating thickness, but from the viewpoint of simplicity and surface gloss or smoothness being imparted by self-leveling, it is preferably 10 seconds to 20 minutes, more preferably 15 seconds to 10 minutes, yet more preferably 30 seconds to 5 minutes, and particularly preferably 30 seconds to 2 minutes.

### -Exposure step-

When an artificial nail is formed by exposure to light, the artificial nail composition of the present invention used preferably comprises (Component B) a polymerizable compound and (Component C) a photopolymerization initiator.

Furthermore, when the artificial nail composition of the present invention comprises (Component D) a solvent in addition to Component B and Component C, it is preferable to carry out drying before carrying out exposure to light.

Examples of light used for exposure include UV and visible light.

The exposure means is not particularly limited; known exposure means may be used, and examples include a UV lamp such as a mercury lamp or a metal halide lamp, a light-emitting diode (LED), and a laser diode (LD).

The exposure time is not particularly limited and is preferably 2 seconds to 5 minutes, more preferably 5 seconds to 3 minutes, and yet more preferably 5 seconds to 2 minutes. Furthermore, exposure may be carried out intermittently, continuously, or pulse light, and any method may be used for exposure.

The method for forming an artificial nail of the present invention may comprise a step of washing or wiping the surface of an obtained artificial nail after the drying or exposure step from the viewpoint of gloss or appearance of the obtained artificial nail and removal of uncured component when exposure has been carried out.

Examples of a washing or wiping method include a method in which wiping is carried out using a wiping substrate such as a wiping sheet or a sponge wipe that are made to contain a solvent such as ethanol, and a method in which washing is carried out with water or a solvent such as ethanol.

Furthermore, a solvent used for washing or wiping is preferably a solvent that does not dissolve an obtained artificial nail.

Moreover, the method for forming an artificial nail of the present invention preferably comprises a step of roughening the surface of a human or animal nail or another artificial nail prior to the coating step, when an artificial nail is formed by exposure to light. With this embodiment, adhesion and durability of an artificial nail (gel nail) obtained by exposure are excellent.

A method for roughening a nail surface is not particularly limited, and it can be carried out by a known method. Preferred examples include a method in which roughening is carried out using a nail sander such as a file.

Furthermore, the method for forming an artificial nail of the present invention may comprise another known step.

### (Method for removing artificial nail)

The method for removing an artificial nail of the present invention comprises a step (removal step) of removing the artificial nail of the present invention by contacting it with a removal liquid.

The removal liquid is preferably an acidic aqueous solution having a pH of no greater than 5.0.

The artificial nail composition of the present invention can be removed by an organic solvent such as acetone, which is a conventional removal liquid, but it can be removed more safely without imposing a burden on the skin and nail by means of an acidic aqueous solution having a pH of no greater than 5.0. A removal liquid suitably used in the present invention preferably comprises an organic/inorganic acid component for making it acidic, water, an optional stabilizer, and an optional surfactant. An embodiment of a preferred removal liquid is explained below.

### <Organic/inorganic acid component>

As the removal liquid, a known inorganic acid (phosphoric acid, hydrochloric acid, etc.) or an organic acid may be used in order to make the aqueous solution acidic. As an acid component used in the removal liquid, an organic acid that is usually contained in food or a food additive is preferably used. Preferred examples of the organic acid include citric acid, gluconic acid, lactic acid, malic acid, an amino acid (aspartic acid, glutamic acid, etc.), and carbonic acid.

### <Water and pH>

The water used in the removal liquid is not particularly limited, and tap water, distilled water, ion-exchanged water, etc. may be used.

The content of the water in the removal liquid is not particularly limited and is preferably 50 to 99.5 mass% relative to the total mass of the removal liquid, more preferably 70 to 99 mass%, and yet more preferably 90 to 99 mass%.

Furthermore, from the viewpoint of cost, transportability, and storage properties, the removal liquid may be a concentrated liquid, and an embodiment in which a removal liquid is formed by diluting this concentrated liquid with water, etc. before use may be employed.

The pH of the removal liquid is, from the viewpoint of the ability to ionize the amino group-containing polymer contained in the artificial nail of the present invention, that is, from the viewpoint of removability, preferably no greater than 5.0, more preferably no greater than 4.0, and yet more preferably no greater than 3.0. Furthermore, the pH of the removal liquid is preferably at least 1.0, and more preferably at least 2.0 from the viewpoint of safety toward the skin and nail.

Furthermore, it is preferable to add an acid salt so as to make a buffer in order to prevent the pH from varying over time or varying due to an external factor such as dilution/concentration, etc. of the liquid. In order to make a buffer, a method in which a salt of the above acid, preferably the sodium salt (sodium citrate, sodium gluconate, sodium carbonate, disodium phosphate) is made to be present at the same time as the acid can be cited, but this should not be construed as being particularly limiting.

### <Stabilizer>

The removal liquid used in the present invention may optionally contain a stabilizer. Addition of a stabilizer can suppress coloration of the liquid over time, the occurrence of corrosion and mold, etc. The stabilizer is not particularly limited, and a known industrially used preservative, food preservative, pharmaceutical preservative, antioxidant/photostabilizer, etc. may be used.

Among them, as the stabilizer, a parahydroxybenzoic acid ester compound is preferable.

The amount of stabilizer added is not particularly limited and is preferably 0.001 to 10 mass% relative to the total mass of the removal liquid, more preferably 0.005 to 5 mass%, and yet more preferably 0.01 to 2 mass%.

From the viewpoint of increasing the removal speed, the removal liquid is preferably heated before use to a degree that does not impair the safety. The heating is preferably to 30°C to 45°C, more preferably to 35°C to 45°C, and particularly preferably to 40°C to 45°C. Furthermore, when an artificial nail is immersed in a removal liquid, the liquid in which it is immersed may be stirred.

The artificial nail of the present invention may be removed easily by contacting it with an acidic aqueous solution having a pH of no greater than 5.0 in place of a conventionally used organic solvent such as acetone.

A contacting method is not particularly limited; an artificial nail may be directly immersed in a removal liquid, an artificial nail may be wrapped with cotton, etc. that is wetted with a removal liquid, or spraying may be carried out using an atomizer or a shower. However, it should not be construed as being limited to these examples.

Furthermore, the method of removing an artificial nail of the present invention preferably comprises a step of damaging the artificial nail surface and/or the extremity so that part of a layer formed with the artificial nail composition of the present invention is exposed on the surface.

A damaging method is not particularly limited, but a nail sander such as a file may be suitably used.

Specific preferred examples of the removal method are shown below. That is, after the artificial nail surface and/or the extremity is optionally and roughly damaged by rubbing with a nail sander to a degree such that part of a layer formed with the artificial nail composition of the present invention is exposed on the surface layer, then immersed in an acidic aqueous solution having a pH of no greater than 5.0, and allowed to stand for about 1 to 5 minutes to thus change the solubility of the artificial nail, the artificial nail is wiped with a cloth, an unwoven cloth, etc., or peeled off with a pushing force using a tool with a spatula shape or a stick shape, thus enabling it to be peeled off very easily and safely without imposing a burden on the finger tip or nail.

In addition, wiping and peeling with a pushing force can be carried out during immersion, and removal can be carried out more rapidly. Furthermore, wiping/peeling may be promoted by applying ultrasonic waves, vibration, etc.

Moreover, the method for removing an artificial nail of the present invention may comprise another known step.

### (Nail art kit)

The nail art kit of the present invention comprises the artificial nail composition of the present invention and a removal liquid, the removal liquid being preferably an acidic aqueous solution having a pH of no greater than 5.0.

Preferred embodiments of the artificial nail composition of the present invention and the removal liquid in the nail art kit of the present invention are the same as those described above.

Furthermore, the nail art kit of the present invention may comprise an optional product in addition to the artificial nail composition and the removal liquid.

Examples include, but are not limited to, an artificial nail composition for coloring, the top, etc. other than the artificial nail composition of the present invention, a nail sander such as a file, a brush or a fine brush such as a flat fine brush for coating the artificial nail composition, exposure equipment such as a UV light, a liquid for wiping or washing, a wipe for wiping, a nail brush, a dust brush, a nail form used for extending a nail, a decorative stone made of an acrylic, glass, metal, or natural stone, a nail sticker, a decorative powder such as glitter or a hologram, a cutter, a spatula, a stick, and a spacer for keeping fingers spaced in order to prevent nails from contacting each other.

### Examples

The present invention is more specifically explained by way of Examples below, but the present invention should not be construed as being limited by the modes of these Examples. Unless otherwise specified, 'parts' and '%' are on a mass basis.

A-1 to A-114 described in the Examples are the same polymers as A-1 to A-114 described above.

### <Synthetic Example 1>

A 1,000 mL three-necked flask equipped with a reflux condenser was charged with 300 g of methyl ethyl ketone and maintained at 65°C under a flow of nitrogen. Subsequently, a mixed liquid of 78.5 g of dimethylaminoethyl methacrylate (Wako Pure Chemical Industries, Ltd.), 71 g of n-butyl methacrylate (Wako Pure Chemical Industries, Ltd.), 4.6 g of dimethyl 2,2'-azobis(2-methylpropionate) (Wako Pure Chemical Industries, Ltd.), and 400 g of methyl ethyl ketone was added thereto dropwise over 3 hours. After completion of the dropwise addition, 4.6 g of dimethyl 2,2'-azobis(2-methylpropionate) (Wako Pure Chemical Industries, Ltd.) was added, and the mixture was heated at 70°C for a further 2 hours. The reaction solution thus obtained was subjected to distillation under reduced pressure, thus giving an 80 mass% solution of A-19. The weight-average molecular weight of the A-19 measured by gel permeation chromatography was 32,000.

### <Synthetic Example 2>

A 1,000 mL three-necked flask equipped with a reflux condenser and a calcium chloride tube was charged with 300 g of methyl ethyl ketone, 84 g of 3-(dimethylamino)-1,2-propanediol (Tokyo Chemical Industry Co., Ltd.), and 60 g of hexamethylene diisocyanate (Tokyo Chemical Industry Co., Ltd.) and maintained at 60°C under a flow of nitrogen. Subsequently, 0.8 g of the inorganic bismuth catalyst Neostann U-600 (Nitto Chemical Industry Co., Ltd.) was added, and heating was carried out for 5 hours while maintaining the temperature at 60°C. The reaction solution thus obtained was added dropwise to 4,000 mL of hexane while stirring, and the resulting mixture was subjected to filtration under aspiration and then drying under reduced pressure, thus giving A-33 as a white solid. The weight-average molecular weight of the A-33 measured by gel permeation chromatography was 22,000.

### <Synthetic Example 3>

A-1 to A-18 and A-20 to A-31 were obtained by the same method as in Synthetic Example 1 except that the starting materials, the amount thereof used, and the amount of polymerization initiator used were changed as appropriate.

### <Synthetic Example 4>

A-32 and A-34 to A-114 were obtained by the same method as in Synthetic Example 2 except that the starting materials, the amount thereof used, and the amount of catalyst initiator used were changed as appropriate.

### Preparation of artificial nail composition

Artificial nail compositions J-1 to J-34 and H-1 to H-6 were obtained by uniformly mixing the various components shown in Table 13 to Table 16 below. The compositions described in Table 13 to Table 15 are artificial nail compositions for radical photocuring, and the compositions described in Table 16 are artificial nail compositions for drying.

**(Table 13)**

| Artificial nail composition | | J-1 | J-2 | J-3 | J-4 | J-5 | J-6 | J-7 | J-8 | J-9 | J-10 |
|---|---|---|---|---|---|---|---|---|---|---|---|
| | A-1 | 55 | - | - | - | - | - | - | - | - | - |
| | A-9 | - | 55 | - | - | - | - | - | - | - | - |
| | A-11 | - | - | 55 | - | - | - | - | - | - | - |
| | A-15 | - | - | - | 55 | - | - | - | - | - | - |
| Polymer | A-19 | - | - | - | - | 55 | - | - | - | - | - |
| | A-20 | - | - | - | - | - | 55 | - | - | - | - |
| | A-21 | - | - | - | - | - | - | 55 | - | - | - |
| | A-22 | - | - | - | - | - | - | - | 55 | - | - |
| | A-23 | - | - | - | - | - | - | - | - | 55 | - |
| | A-32 | - | - | - | - | - | - | - | - | - | 55 |
| Polymerizable compound | B-101 | 5 | 5 | 5 | 5 | 5 | 5 | 5 | 5 | 5 | 5 |
| | B-103 | 12 | 12 | 12 | 12 | 12 | 12 | 12 | 12 | 12 | 12 |
| Photopolymerization initiator | B-201 | 1 | 1 | 1 | 1 | 1 | 1 | 1 | 1 | 1 | 1 |
| Organic solvent | Methyl ethyl ketone | 12 | 12 | 12 | 12 | 12 | 12 | 12 | 12 | 12 | 12 |
| | Isopropanol | 15 | 15 | 15 | 15 | 15 | 15 | 15 | 15 | 15 | 15 |

**(Table 14)**

| Artificial nail composition | | J-11 | J-12 | J-13 | J-14 | J-15 | J-16 | H-1 | H-2 | H-3 | H-4 |
|---|---|---|---|---|---|---|---|---|---|---|---|
| | A-19 | - | - | 25 | 35 | 55 | 55 | - | - | - | - |
| | A-36 | 55 | - | - | - | - | - | - | - | - | - |
| Polymer | A-37 | - | 55 | - | - | - | - | - | - | - | - |
| | HA-1 | - | - | - | - | - | 10 | 55 | 55 | - | - |
| | HA-2 | - | - | - | - | - | - | - | - | 55 | - |
| | HA-3 | - | - | - | - | - | - | - | - | - | 55 |
| Polymerizable compound | B-101 | 5 | 5 | 15 | 24 | 5 | 5 | 5 | - | 5 | 5 |
| | B-102 | - | - | - | - | - | - | - | 20 | - | - |
| | B-103 | 12 | 12 | 32 | 28 | 12 | 12 | 12 | 12 | 12 | 12 |
| Photo-polymerization initiator | B-201 | 1 | 1 | 1 | 1 | - | 1 | 1 | 1 | 1 | 1 |
| | B-202 | - | - | - | - | 1 | - | - | - | - | - |
| Organic solvent | Methyl ethyl ketone | 12 | 12 | 12 | 5 | 12 | 7 | 12 | 5 | 12 | 12 |
| | Isopropanol | 15 | 15 | 15 | 7 | 15 | 10 | 15 | 7 | 15 | 15 |

**(Table 15)**

| Artificial nail composition | | J-17 | J-18 | J-19 | J-20 | J-21 | J-22 | J-23 |
|---|---|---|---|---|---|---|---|---|
| Polymer | A-19 | 55 | 55 | 55 | 55 | 55 | 55 | 55 |
| | B-101 | - | - | - | - | - | - | 5 |
| | B-103 | 12 | 12 | 12 | 12 | 12 | 12 | 12 |
| | B-104 | 5 | - | - | - | - | - | - |
| Polymerizable compound | B-105 | - | 5 | - | - | - | - | - |
| | B-106 | - | - | 5 | - | - | - | - |
| | B-107 | - | - | - | 5 | - | - | - |
| | B-108 | - | - | - | - | 5 | - | - |
| | B-109 | | | - | - | - | 5 | - |
| Photopoly-merization initiator | B-201 | 1 | 1 | 1 | 1 | 1 | 1 | - |
| | B-203 | - | - | - | - | - | - | 1 |
| Organic solvent | Methyl ethyl ketone | 12 | 12 | 12 | 12 | 12 | 12 | 12 |
| | Isopropanol | 15 | 15 | 15 | 15 | 15 | 15 | 15 |

**(Table 16)**

| Artificial nail composition | | J-24 | J-25 | J-26 | J-27 | J-28 | J-29 | J-30 | J-31 | J-32 | J-33 | J-34 | H-5 | H-6 |
|---|---|---|---|---|---|---|---|---|---|---|---|---|---|---|
| | A-1 | 60 | - | - | - | - | - | - | - | - | - | - | - | - |
| | A-19 | - | 60 | - | - | - | - | - | 25 | 60 | 60 | 60 | - | - |
| | A-20 | - | - | 60 | - | - | - | - | - | - | - | - | - | - |
| | A-21 | - | - | - | 60 | - | - | - | - | - | - | - | - | - |
| Polymer | A-22 | - | - | - | - | 60 | - | - | - | - | - | - | - | - |
| | A-23 | - | - | - | - | - | 60 | - | - | - | - | - | - | - |
| | A-32 | - | - | - | - | - | - | 60 | - | - | - | - | - | - |
| | HA-1 | - | - | - | - | - | - | - | - | 10 | - | - | 60 | - |
| | HA-3 | - | - | - | - | - | - | - | - | - | - | - | - | 60 |
| Organic solvent | Methyl ethyl ketone | 28 | 28 | 28 | 28 | 28 | 28 | 28 | 53 | 23 | 28 | 28 | 28 | 28 |
| | Isopropanol | 12 | 12 | 12 | 12 | 12 | 12 | 12 | 22 | 7 | - | - | 12 | 12 |
| | Butyl acetate | - | - | - | - | - | - | - | - | - | 12 | - | - | - |
| | MFG | - | - | - | - | - | - | - | - | - | - | 12 | - | - |

The abbreviations given in Table 13 to Table 16 are as follows.

Furthermore, methyl ethyl ketone (boiling point 80°C), isopropanol (boiling point 82.4°C), butyl acetate (boiling point 126°C), and MFG (propylene glycol monomethyl ether, boiling point 120°C), which were used as solvents, were all manufactured by Tokyo Chemical Industry Co., Ltd.

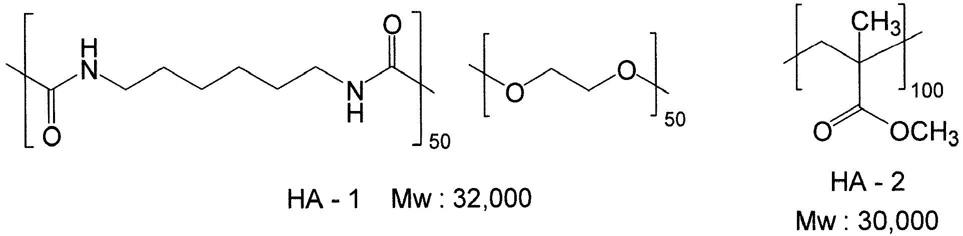

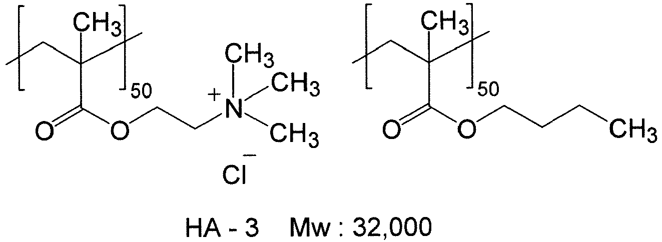

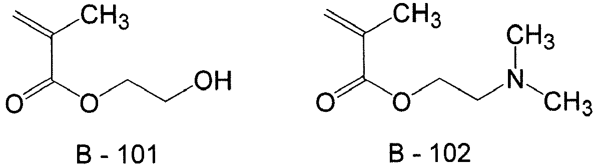

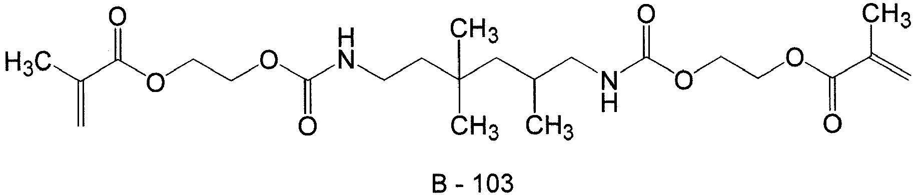

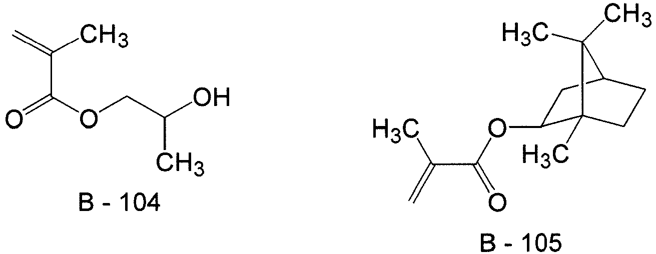

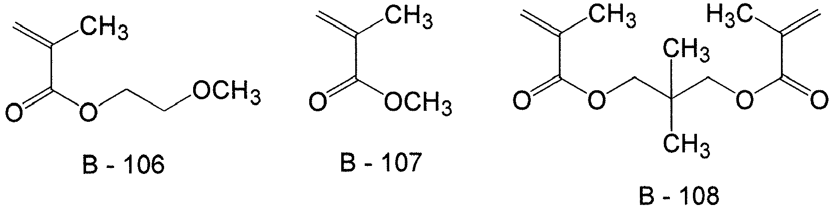

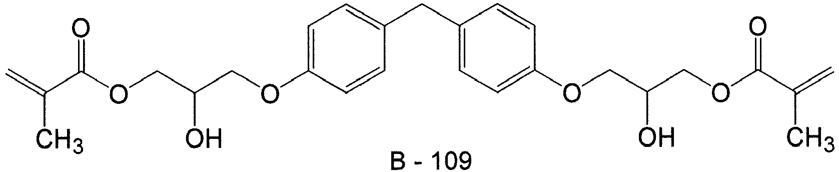

In addition, the B-101 and B-105 to B-108 used were manufactured by Tokyo Chemical Industry Co., Ltd., the B-102 used was manufactured by Wako Pure Chemical Industries, Ltd., the B-104 used was Light-Ester HPO (N) manufactured by Kyoeisha Chemical Co., Ltd., and the B-109 used was manufactured by Sigma Aldrich.

The B-103 used was synthesized by reacting 1 molar equivalent of the corresponding diisocyanate compound with 2 molar equivalents of 2-hydroxyethyl methacrylate.

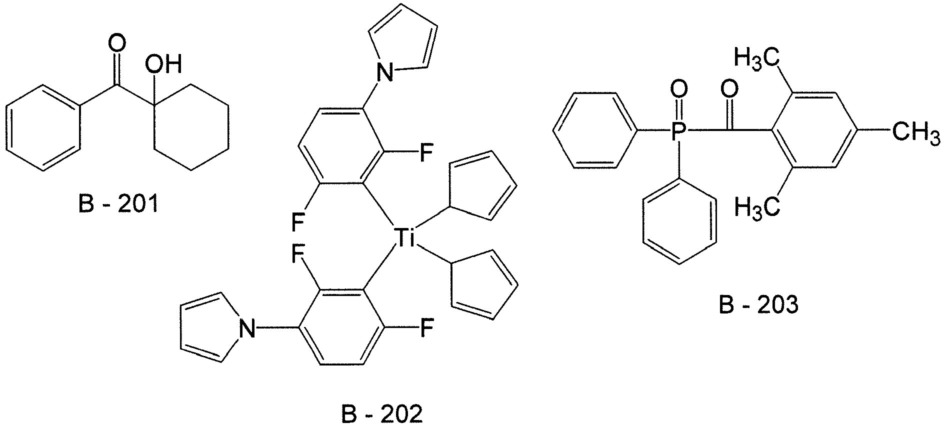

B-201 was IRGACURE 184 manufactured by BASF Japan, B-202 was IRGACURE 784 manufactured by BASF Japan, and B-203 was diphenyl(2,4,6-trimethylbenzoyl)phosphine oxide (Wako Pure Chemical Industries, Ltd.).

### Preparation of removal liquid

Removal liquids E-1 to E-6 were obtained by uniformly mixing the various components shown in Table 17 below.

**(Table 17)**

| Removal liquid | | E-1 | E-2 | E-3 | E-4 | E-5 | E-6 |
|---|---|---|---|---|---|---|---|
| Organic/inorganic acid component | Citric acid | 2.7 | 2.0 | 1.2 | - | - | 2.7 |
| | Trisodium citrate dihydrate | 0.3 | 1.3 | 2.5 | - | - | 0.3 |
| | Glycolic acid | - | - | - | - | - | - |
| | Phosphoric acid | - | - | - | - | - | - |
| Stabilizer | Benzyl parahydroxybenzoate | - | - | - | - | - | 1 |
| Surfactant | Lauryldimethylaminoacetic acid betaine | - | - | - | - | - | 1 |
| Other component | Tap water | 297 | 296.7 | 296.3 | * | * | 295 |
| pH of liquid | | 2.6 | 3.5 | 4.7 | 2.6 | 2.6 | 2.6 |

| | | | | | | | |
|---|---|---|---|---|---|---|---|
| *: removal liquids E-4 and E-5 were prepared by combining tap water and the organic/inorganic acid components described in Table 17 so as to give a pH of 2.6. | | | | | | | |

Details of the reagents used in the removal liquids are as follows.
Citric acid (Tokyo Chemical Industry Co., Ltd.)
Trisodium citrate dihydrate (Wako Pure Chemical Industries, Ltd.)
Glycolic acid (Tokyo Chemical Industry Co., Ltd.)
Phosphoric acid (Wako Pure Chemical Industries, Ltd.)
Benzyl parahydroxybenzoate (Tokyo Chemical Industry Co., Ltd.)
Lauryldimethylaminoacetic acid betaine (Amphitol 20BS, Kao Corporation)
Formation of artificial nail

### <Formation Example 1>

A plastic substrate shaped into a nail shape was coated using a flat brush with a fixed amount of the artificial nail composition obtained and irradiated using a UV lamp (36 W) for 2 minutes. Subsequently, the surface was washed with ethanol, and when the artificial nail thus formed was then visually examined, it had completely solidified. The film thickness of the artificial nail at this time was measured and was found to be about 100 µm (±10 µm).

### <Formation Example 2>

An artificial nail having a film thickness of about 100 µm (±10 µm) formed in the same way as in Formation Example 1 was coated with the commercially available Calgel #CG-03 fresh pink (Moga·Brook Co., Ltd.) as a color layer using a flat brush, and irradiation was carried out using a UV lamp (36 W) for 2 minutes. After an uncured portion was wiped with ethanol, coating as a top layer with a commercially available top gel (Moga·Brook Co., Ltd.) using a flat brush was carried out and irradiation was carried out in the same manner with a UV lamp (36 W) for 2 minutes. When the artificial nail thus formed was visually examined, it had completely solidified. The total film thickness of the color layer and the top layer was about 1,000 µm.

### <Formation Example 3>

A plastic substrate shaped into a nail shape was coated using a flat brush with a fixed amount of the artificial nail composition obtained and dried naturally for 1 minute after coating. Subsequently, the surface was washed with ethanol, and when the artificial nail was then visually examined, it had completely solidified. The film thickness at this time was measured and was found to be about 100 µm (±10 µm).

### <Formation Example 4>

An artificial nail having a film thickness of about 100 µm (±10 µm) formed in the same manner as in Formation Example 3 was coated with the commercially available Calgel #CG-03 fresh pink (Moga·Brook Co., Ltd.) as a color layer using a flat brush, and irradiation was carried out using a UV lamp (36 W) for 2 minutes. After an uncured portion was wiped with ethanol, coating as a top layer with a commercially available top gel (Moga·Brook Co., Ltd.) using a flat brush was carried out and irradiation was carried out in the same manner with a UV lamp (36 W) for 2 minutes. When the artificial nail thus formed was visually examined, it had completely solidified. The total film thickness of the color layer and the top layer was about 1,000 µm.

### Evaluation of artificial nail

### <Gloss>

The gloss of the artificial nail formed was visually evaluated using the criteria below under the light of a fluorescent lamp.

### (Evaluation criteria)

A: the fluorescent lamp was seen on the nail surface, and the image could be clearly recognized.
B: the fluorescent lamp was seen on the nail surface, and the image could be fairly clearly recognized.
C: the fluorescent lamp was seen on the nail surface, but the image could not be clearly recognized.
D: the fluorescent lamp was not seen on the nail surface.

### <Removability-1>

The artificial nail obtained was immersed in a removal liquid described in the Table below at 40°C for 15 seconds and wiped 20 times using kitchen paper soaked with the removal liquid. This immersion-wiping operation was repeated 10 times, and following this the film thickness (µm) of the remaining artificial nail was evaluated.

### <Removability-2>

The surface and extremity of the artificial nail obtained was damaged using a file (nail sander). Subsequently, it was immersed in a removal liquid described in the Table below at 40°C for 2 minutes; after 2 minutes had elapsed, a peeling operation was carried out using a wooden spatula while it was immersed, and the ease of removal from a substrate was evaluated using the evaluation criteria below.

### -Evaluation criteria-

A: could be easily removed.
B: could be removed.
C: could be removed, but slight degree of film remained that could be wiped off using removal liquid.
D: could not be removed.

### <Adhesion-1>

The uppermost layer of the artificial nail obtained was scraped using an HB pencil with a fixed load, and the presence or absence of peel-off and the presence or absence of scratching were evaluated using the evaluation criteria below.

### -Evaluation criteria-

A: neither scratching nor peel-off occurred.
B: peel-off did not occur, but some scratching occurred.
C: peel-off did not occur, but scratching occurred.
D: peel-off occurred.

### <Water resistance-1>

The artificial nail obtained was placed in tap water at 45°C (pH 6.4), and stirring was carried out at 200 rpm for 5 hours. The artificial nail was taken out after stirring, the surface state was visually examined, and evaluation was carried out using the evaluation criteria below.
A: neither scratching nor peel-off occurred.
B: peel-off did not occur, but some scratching occurred.
C: peel-off did not occur, but scratching occurred.
D: peel-off occurred.

### <Evaluation when aged artificial nail composition was used>

The artificial nail composition was charged into a brown bottle and then stored in a temperature-controlled room at 40°C for 5 days. After storage, an artificial nail was formed by the same method as the above method, and removability, adhesion, water resistance, and gloss were evaluated.

### (Examples 1 to 24 and Comparative Examples 1 to 7)

### <Evaluation of artificial nail formed by radical curing>

The artificial nail produced in Formation Example 1 was subjected to evaluation in terms of gloss, removability-1, adhesion-1, and water resistance-1, and the same evaluation was carried out using an aged artificial nail composition. The results are shown in Table 18. Furthermore, the results of evaluating removability-1 by variously changing the removal liquid are shown in Table 19.

**(Table 18)**

| | Artificial nail composition | Formation method | Use of artificial nail composition immediately after preparation | | | | | | Use of aged artificial nail composition | | | | | |
|---|---|---|---|---|---|---|---|---|---|---|---|---|---|---|
| | | | Gloss | Removability-1 | | | Adhesion-1 | Water resistance-1 | Gloss | Removability-1 | | | Adhesion-1 | Water resistance-1 |
| | | | | Removal liquid E-1 | Removal liquid E-2 | Removal liquid E-3 | | | | Removal liquid E-1 | Removal liquid E-2 | Removal liquid E-3 | | |
| Ex. 1 | J-1 | | B | 0 | 0 | 0 | A | C | B | 0 | 0 | 0 | A | C |
| Ex. 2 | J-2 | | B | 1 | 3 | 7 | A | A | B | 1 | 3 | 8 | A | A |
| Ex. 3 | J-3 | | B | 1 | 3 | 8 | A | A | B | 1 | 3 | 9 | A | A |
| Ex. 4 | J-4 | | B | 5 | 12 | 21 | B | B | B | 6 | 14 | 24 | B | B |
| Ex. 5 | J-5 | | B | 0 | 0 | 0 | A | A | B | 0 | 0 | 0 | A | A |
| Ex. 6 | J-6 | | B | 2 | 4 | 9 | B | A | B | 2 | 5 | 10 | B | A |
| Ex. 7 | J-7 | | B | 2 | 6 | 12 | B | A | B | 2 | 7 | 14 | B | A |
| Ex. 8 | J-8 | | B | 0 | 0 | 2 | B | B | B | 0 | 0 | 2 | B | B |
| Ex. 9 | J-9 | | B | 0 | 0 | 2 | B | C | B | 0 | 0 | 2 | B | C |
| Ex. 10 | J-10 | | B | 0 | 0 | 2 | A | A | B | 0 | 0 | 2 | A | A |
| Ex. 11 | J-11 | | B | 0 | 0 | 0 | A | C | B | 0 | 0 | 0 | A | C |
| Ex. 12 | J-12 | | B | 2 | 4 | 9 | A | A | B | 2 | 5 | 10 | A | A |
| Ex. 13 | J-13 | Formation Ex. 1 | B | 2 | 4 | 7 | B | A | B | 2 | 5 | 8 | B | A |
| Ex. 14 | J-14 | | B | 3 | 6 | 12 | A | A | B | 3 | 7 | 14 | A | A |
| Ex. 15 | J-15 | | B | 0 | 0 | 0 | A | A | B | 0 | 0 | 0 | A | A |
| Ex. 16 | J-16 | | A | 2 | 4 | 6 | A | A | C | 2 | 5 | 7 | A | A |
| Ex. 17 | J-17 | | B | 0 | 0 | 0 | A | A | B | 0 | 0 | 0 | A | A |
| Ex. 18 | J-18 | | B | 0 | 0 | 1 | A | A | B | 0 | 0 | 1 | A | A |
| Ex. 19 | J-19 | | B | 0 | 0 | 1 | A | A | B | 0 | 0 | 1 | A | A |
| Ex. 20 | J-20 | | B | 0 | 1 | 2 | A | A | B | 0 | 1 | 2 | A | A |
| Ex. 21 | J-21 | | B | 0 | 2 | 3 | A | B | B | 0 | 2 | 3 | A | B |
| Ex. 22 | J-22 | | B | 0 | 2 | 4 | A | A | B | 0 | 2 | 5 | A | A |
| Ex. 23 | J-23 | | B | 0 | 1 | 4 | A | A | B | 0 | 0 | 0 | A | A |
| Comp. Ex. 1 | H-1 | | B | 100 | 100 | 100 | A | A | C | 100 | 100 | 100 | A | A |
| Comp. Ex. 2 | H-2 | | C | 0 | 0 | 4 | B | B | D | 20 | 32 | 45 | C | C |
| Comp. Ex. 3 | H-3 | | B | 100 | 100 | 100 | A | A | B | 100 | 100 | 100 | B | B |
| Comp. Ex. 4 | H-4 | | C | 0 | 0 | 8 | C | D | C | 0 | 6 | 13 | C | D |

**(Table 19)**

| | Artificial nail composition | Formation method | Removability-1 | | | | | |
|---|---|---|---|---|---|---|---|---|
| | | | Removal liquid E-1 | Removal liquid E-2 | Removal liquid E-3 | Removal liquid E-4 | Removal liquid E-5 | Removal liquid E-6 |
| Example 24 | J-5 | Formation Example 1 | 0 | 0 | 0 | 4 | 4 | 0 |
| Comparative Example 5 | H-1 | | 100 | 100 | 100 | 100 | 100 | 100 |
| Comparative Example 6 | H-2 | | 0 | 0 | 4 | 12 | 16 | 0 |
| Comparative Example 7 | H-4 | | 0 | 0 | 0 | 12 | 10 | 0 |

It can be seen from the results of Table 18 and Table 19 that the artificial nail obtained from the artificial nail composition of the present invention can easily be removed using an acid aqueous solution even when it is cured by exposure to light, and high adhesion can be maintained. It can also be seen that there is little degradation of performance with the artificial nail composition of the present invention even after being aged.

### (Examples 25 to 29 and Comparative Examples 8 to 10)

### <Evaluation of artificial nail formed by radical curing and having upper layer provided>

The artificial nail produced in Formation Example 2 was evaluated in terms of gloss, removability-2, adhesion-1, and water resistance-1, and an aged artificial nail composition was subjected to the same evaluation. The results are shown in Table 20.

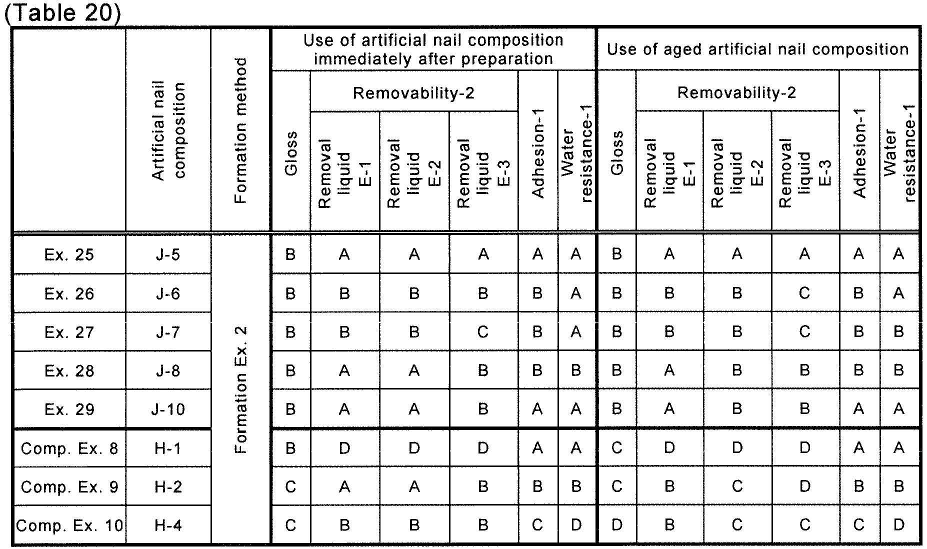

It can be seen from the results of Table 20 that the artificial nail composition of the present invention can be cured by exposure to light, an artificial nail formed by radically curing the artificial nail composition of the present invention can easily be peeled by pushing using an acid aqueous solution even when it further has an upper layer, and high adhesion can be maintained. It can be seen that there is little degradation of performance even after aging.

### (Examples 30 to 40 and Comparative Examples 11 and 12)

### <Evaluation of artificial nail formed by drying.

The artificial nail produced in Formation Example 3 was evaluated in terms of gloss, removability-1, adhesion-1, and water resistance-1, and an aged artificial nail composition was subjected to the same evaluations. The results are shown in Table 21.

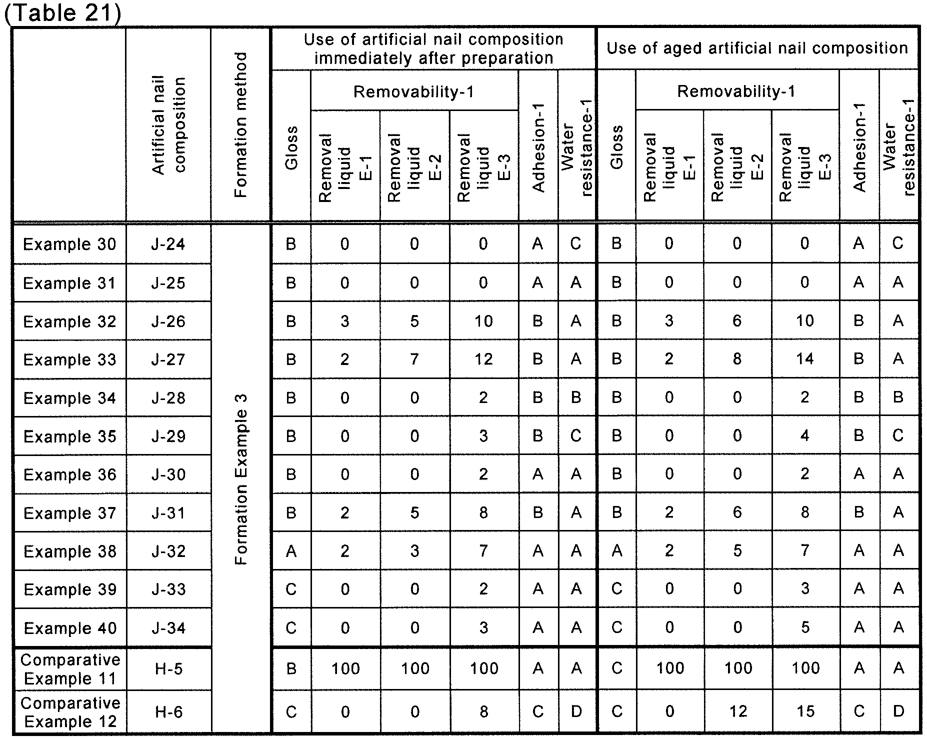

It can be seen from the results of Table 21 that an artificial nail formed by drying and curing the artificial nail composition of the present invention can be easily removed using an acid aqueous solution, and high adhesion can be maintained. It can be seen that there is little degradation of performance even after aging.

### (Examples 41 and 42 and Comparative Examples 13 and 14)

### <Evaluation of artificial nail formed by drying and having upper layer provided>

An artificial nail produced in Formation Example 4 was evaluated in terms of gloss, removability-2, adhesion-1, and water resistance-1, and an aged artificial nail composition was subjected to the same evaluation. The results are shown in Table 22.

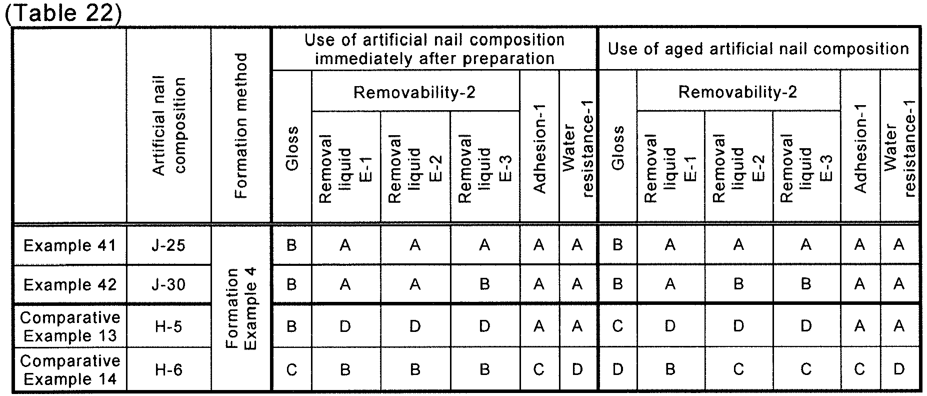

It can be seen from the results of Table 22 that an artificial nail formed by drying and curing the artificial nail composition of the present invention can be easily peeled by pushing using an acid aqueous solution even when it has an upper layer, and high adhesion can be maintained. It can be seen that there is little degradation of performance even after aging.

### Preparation of artificial nail composition

The various components shown in Table 23 to Table 26 below were uniformly mixed, thus giving artificial nail compositions J-35 to J-70 and H-7 to H-10. The compositions described in Table 23 to Table 26 are artificial nail compositions for radical photocuring.

**(Table 23)**

| Artificial nail composition | | J-35 | J-36 | J-37 | J-38 | J-39 | J-40 | J-41 | J-42 | J-43 | J-44 |
|---|---|---|---|---|---|---|---|---|---|---|---|
| | A-1 | 15 | - | - | - | - | - | - | - | - | - |
| | A-19 | - | 15 | - | - | - | - | - | - | - | - |
| | A-35 | - | - | 15 | - | - | - | - | - | - | - |
| | A-38 | - | - | - | 15 | - | - | - | - | - | - |
| Polymer | A-83 | - | - | - | - | 15 | - | - | - | - | - |
| | A-84 | - | - | - | - | - | 15 | - | - | - | - |
| | A-39 | - | - | - | - | - | - | 15 | - | - | - |
| | A-33 | - | - | - | - | - | - | - | 15 | - | - |
| | A-40 | - | - | - | - | - | - | - | - | 15 | - |
| | A-51 | - | - | - | - | - | - | - | - | - | 15 |
| Polymerizable compound | B-104 | 54 | 54 | 54 | 54 | 54 | 54 | 54 | 54 | 54 | 54 |
| | B-114 | 25 | 25 | 25 | 25 | 25 | 25 | 25 | 25 | 25 | 25 |
| Photo-polymerization initiator | B-201 | 3 | 3 | 3 | 3 | 3 | 3 | 3 | 3 | 3 | 3 |
| | B-203 | 3 | 3 | 3 | 3 | 3 | 3 | 3 | 3 | 3 | 3 |

**(Table 24)**

| Artificial nail composition | | J-45 | J-46 | J-47 | J-48 | J-49 | J-50 | J-51 | J-52 | J-53 | J-54 |
|---|---|---|---|---|---|---|---|---|---|---|---|
| | A-52 | 15 | - | - | - | - | - | - | - | - | - |
| | A-53 | - | 15 | - | - | - | - | - | - | - | - |
| | A-54 | - | - | 15 | - | - | - | - | - | - | - |
| | A-55 | - | - | - | 15 | - | - | - | - | - | - |
| Polymer | A-56 | - | - | - | - | 15 | - | - | - | - | - |
| | A-79 | - | - | - | - | - | 15 | - | - | - | - |
| | A-80 | - | - | - | - | - | - | 15 | - | - | - |
| | A-57 | - | - | - | - | - | - | - | 15 | - | - |
| | A-58 | - | - | - | - | - | - | - | - | 15 | - |
| | A-59 | - | - | - | - | - | - | - | - | - | 15 |
| Polymeri-zable compound | B-104 | 54 | 54 | 54 | 54 | 54 | 54 | 54 | 54 | 54 | 54 |
| | B-114 | 25 | 25 | 25 | 25 | 25 | 25 | 25 | 25 | 25 | 25 |
| Photo-polymerization initiator | B-201 | 3 | 3 | 3 | 3 | 3 | 3 | 3 | 3 | 3 | 3 |
| | B-203 | 3 | 3 | 3 | 3 | 3 | 3 | 3 | 3 | 3 | 3 |

**(Table 25)**

| Artificial nail composition | | J-55 | J-56 | J-57 | J-58 | J-59 | J-60 | J-61 | J-62 | J-63 |
|---|---|---|---|---|---|---|---|---|---|---|
| | A-60 | 15 | - | - | - | - | - | - | - | - |
| | A-71 | - | 15 | - | - | - | - | - | - | - |
| | A-72 | - | - | 15 | - | - | - | - | - | - |
| | A-75 | - | - | - | 15 | - | - | - | - | - |
| Polymer | A-61 | - | - | - | - | 15 | - | - | - | - |
| | A-65 | - | - | - | - | - | 15 | - | - | - |
| | A-90 | - | - | - | - | - | - | 15 | - | - |
| | A-91 | - | - | - | - | - | - | - | 15 | - |
| | A-94 | - | - | - | - | - | - | - | - | 15 |
| Polymerizable compound | B-104 | 54 | 54 | 54 | 54 | 54 | 54 | 54 | 54 | 54 |
| | B-114 | 25 | 25 | 25 | 25 | 25 | 25 | 25 | 25 | 25 |
| Photo-polymerization initiator | B-201 | 3 | 3 | 3 | 3 | 3 | 3 | 3 | 3 | 3 |
| | B-203 | 3 | 3 | 3 | 3 | 3 | 3 | 3 | 3 | 3 |

**(Table 26)**

| Artificial nail composition | | J-64 | J-65 | J-66 | J-67 | J-68 | J-69 | J-70 | H-7 | H-8 | H-9 | H-10 |
|---|---|---|---|---|---|---|---|---|---|---|---|---|
| Polymer | A-54 | 15 | 15 | 15 | 15 | 15 | 15 | 15 | - | - | - | - |
| | HA-1 | - | - | - | - | - | - | - | 15 | - | - | 47 |
| | HA-2 | - | - | - | - | - | - | - | - | 15 | - | - |
| | HA-3 | - | - | - | | - | - | - | - | - | 40 | - |
| | B-104 | 54 | 54 | 54 | - | - | - | - | 30 | 30 | 54 | 47 |
| | B-102 | - | - | - | - | - | - | - | 39 | 39 | - | - |
| | B-103 | - | - | 25 | - | - | - | - | - | - | - | - |
| | B-101 | - | - | - | 54 | - | - | - | - | - | - | - |
| Polymerizable compound | B-110 | - | - | - | - | 27 | - | - | - | - | - | - |
| | B-111 | - | - | - | - | - | 27 | - | - | - | - | - |
| | B-112 | - | - | - | - | - | - | 54 | - | - | - | - |
| | B-113 | - | - | - | - | 27 | 27 | - | - | - | - | - |
| | B-114 | 25 | - | - | - | 25 | 25 | 25 | - | - | - | - |
| | B-115 | - | 25 | - | - | - | - | - | - | - | - | - |
| Photopolymerization initiator | B-201 | - | 3 | 3 | 3 | 3 | 3 | 3 | 3 | 3 | 3 | 3 |
| | B-203 | 6 | 3 | 3 | 3 | 3 | 3 | 3 | 3 | 3 | 3 | 3 |

The abbreviations in Table 23 to Table 26 other than those described above are as follows. 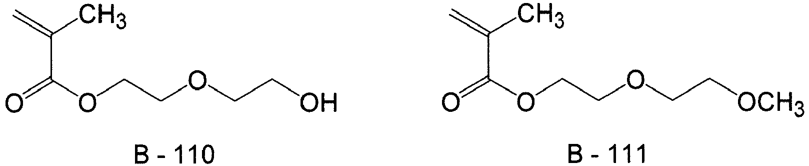 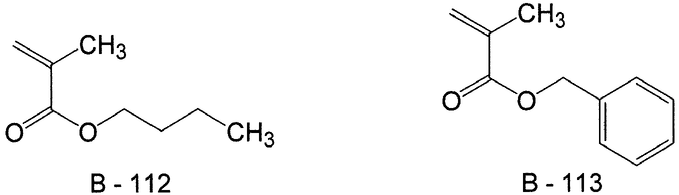 B-114: polyurethane methacrylate compound (UV-3310B, The Nippon Synthetic Chemical Industry Co., Ltd.)
B-115: polyurethane methacrylate compound (UV-3300B, The Nippon Synthetic Chemical Industry Co., Ltd.)

In addition, B-110 was Blemmer PE90 manufactured by NOF Corporation, B-111 was Blemmer PME100 manufactured by NOF Corporation, and B-112 and B-113 were both manufactured by Tokyo Chemical Industry Co., Ltd.

### (Examples 43 to 78 and Comparative Examples 15 to 18)

### <Evaluation of artificial nail formed by radical curing and having upper layer provided>

The artificial nail produced in Formation Example 2 was evaluated in terms of gloss, removability-2, adhesion-1, and water resistance-1, and an aged artificial nail composition was subjected to the same evaluation. The results are shown in Table 27.

**(Table 27)**

| | Artificial nail composition | Formation method | Use of gel composition immediately after preparation | | | | | | Use of aged gel composition | | | | | |
|---|---|---|---|---|---|---|---|---|---|---|---|---|---|---|
| | | | Gloss | Removability-2 | | | Adhesion-1 | Water resistance-1 | Gloss | Removability-2 | | | Adhesion-1 | Water resistance-1 |
| | | | | Removal Liq. | Removal Liq. | Removal Liq. | | | | Removal Liq. | Removal Liq. | Removal Liq. | | |
| | | | | E-1 | E-2 | E-3 | | | | E-1 | E-2 | E-3 | | |
| Ex. 43 | J-35 | | A | A | A | B | B | B | A | A | A | B | B | B |
| Ex. 44 | J-36 | | A | A | A | B | B | A | A | A | A | B | B | A |
| Ex. 45 | J-37 | | B | A | A | B | B | A | B | A | A | B | B | A |
| Ex. 46 | J-38 | | B | A | A | B | B | A | B | A | A | B | B | A |
| Ex. 47 | J-39 | | B | A | A | B | A | B | B | A | A | B | A | B |
| Ex. 48 | J-40 | | B | A | B | B | A | A | B | A | B | B | A | A |
| Ex. 49 | J-41 | | B | A | A | B | B | B | B | A | A | B | B | B |
| Ex. 50 | J-42 | | B | A | A | B | C | B | B | A | A | B | C | B |
| Ex. 51 | J-43 | | B | A | A | B | B | B | B | A | A | B | B | B |
| Ex. 52 | J-44 | | B | A | A | A | B | B | B | A | A | A | B | B |
| Ex. 53 | J-45 | | B | A | A | A | B | B | B | A | A | A | B | B |
| Ex. 54 | J-46 | | B | A | A | A | B | B | B | A | A | A | B | B |
| Ex. 55 | J-47 | | B | A | A | A | A | A | B | A | A | A | A | A |
| Ex. 56 | J-48 | | B | A | A | A | A | B | B | A | A | A | A | B |
| Ex. 57 | J-49 | | B | A | A | B | A | A | B | A | A | B | A | A |
| Ex. 58 | J-50 | | B | A | A | A | A | A | B | A | A | A | A | A |
| Ex. 59 | J-51 | | B | A | A | A | A | A | B | A | A | A | A | A |
| Ex. 60 | J-52 | | B | A | A | A | A | A | B | A | B | B | B | B |
| Ex. 61 | J-53 | Formation Ex. 2 | B | A | A | A | A | B | B | A | B | B | B | B |
| Ex. 62 | J-54 | | B | A | A | B | A | A | B | A | B | C | B | B |
| Ex. 63 | J-55 | | B | A | A | A | B | B | B | A | A | A | B | B |
| Ex. 64 | J-56 | | B | A | B | B | A | A | B | A | B | B | A | A |
| Ex. 65 | J-57 | | B | B | B | B | A | A | B | B | B | B | A | A |
| Ex. 66 | J-58 | | B | A | B | B | B | A | B | A | B | B | B | A |
| Ex. 67 | J-59 | | B | A | A | B | B | B | B | A | A | B | B | B |
| Ex. 68 | J-60 | | B | A | A | B | B | A | B | A | A | B | B | A |
| Ex. 69 | J-61 | | B | A | A | B | B | B | B | A | B | B | B | B |
| Ex. 70 | J-62 | | B | A | A | B | A | B | B | A | B | B | B | B |
| Ex. 71 | J-63 | | B | A | A | B | B | B | B | A | B | B | B | B |
| Ex. 72 | J-64 | | B | A | A | A | A | A | B | A | A | A | A | A |
| Ex. 73 | J-65 | | B | A | A | B | B | A | B | A | A | B | B | B |
| Ex. 74 | J-66 | | B | A | B | B | B | B | B | A | B | B | B | B |
| Ex. 75 | J-67 | | B | A | A | A | A | A | B | A | A | A | A | A |
| Ex. 76 | J-68 | | B | A | A | A | B | A | B | A | A | A | B | A |
| Ex. 77 | J-69 | | B | A | A | A | B | A | B | A | A | A | B | A |
| Ex. 78 | J-70 | | B | A | A | A | B | A | B | A | A | A | B | A |
| Comp. Ex. 15 | H-7 | | B | B | B | C | C | C | B | C | C | C | D | D |
| Comp. Ex. 16 | H-8 | | B | B | B | C A | C | C | B | C | C | C | D | D |
| Comp. Ex. 17 | H-9 | | B | A | A | | D | D | B | A | B | C | D | D |
| Comp. Ex. 18 | H-10 | | B | D | D | D | A | A | B | D | D | D | A | A |

It can be seen from the results of Table 27 that the artificial nail composition of the present invention is cured by exposure to light, an artificial nail formed by radically curing the artificial nail composition of the present invention can easily be peeled by pushing using an acid aqueous solution even when it further has an upper layer, and high adhesion can be maintained. It can be seen that there is little degradation of performance even after aging.

### (Examples 79 to 92 and Comparative Examples 19 to 22)

### <Evaluation of artificial nail formed by radical curing>

The artificial nail produced in Formation Example 1 was evaluated in terms of gloss, removability-1, adhesion-1, and water resistance-1, and an aged artificial nail composition was subjected to the same evaluation. The results are shown in Table 28. The results of evaluating removability-1 by variously changing the removal liquids are shown in Table 29.

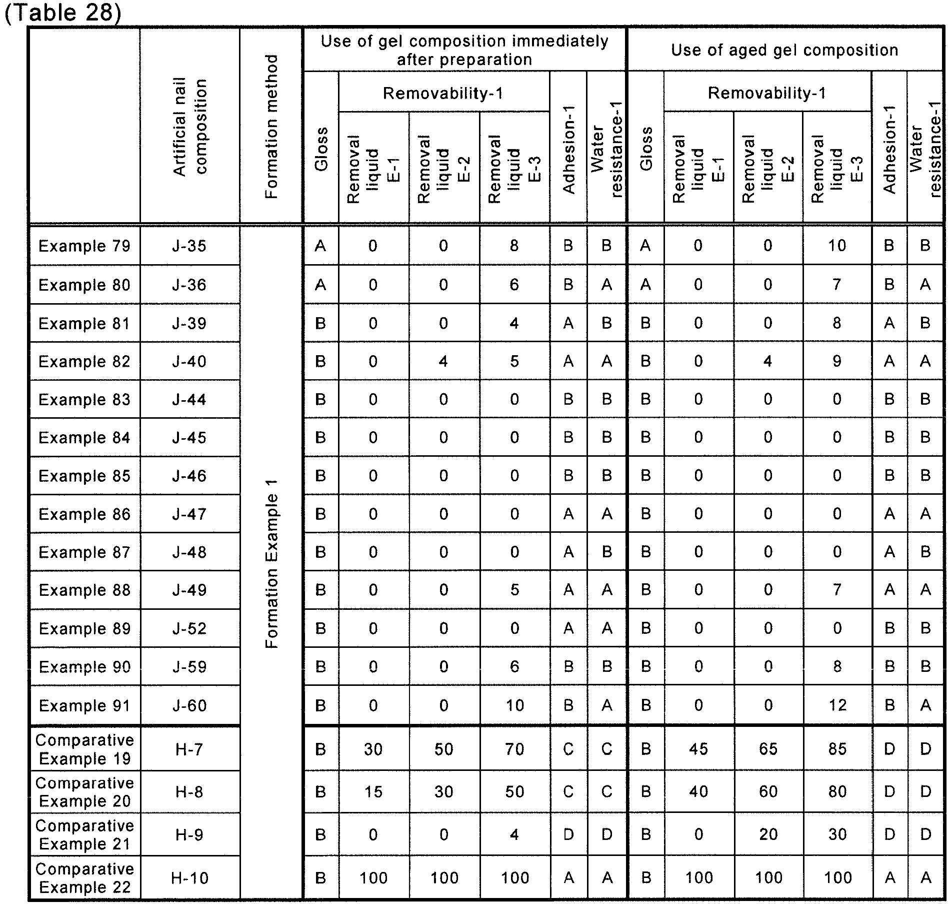

**(Table 29)**

| | Artificial nail composition | Formation method | Removability-1 | | | | | |
|---|---|---|---|---|---|---|---|---|
| | | | Removal liquid E-1 | Removal liquid E-2 | Removal liquid E-3 | Removal liquid E-4 | Removal liquid E-5 | Removal liquid E-6 |
| Example 92 | J-47 | Formation Example 1 | 0 | 0 | 0 | 3 | 6 | 0 |

It can be seen from the results of Table 28 and Table 29 that the artificial nail obtained with the artificial nail composition of the present invention can be easily removed using an acid aqueous solution even when it is cured by exposure to light, and high adhesion can be maintained. It can also be seen that the artificial nail composition of the present invention has little degradation of performance even after being aged.

### Preparation of artificial nail composition

The various components shown in Table 30 to Table 32 below were uniformly mixed, thus giving artificial nail compositions J-71 to J-94 and H-11 to H-14. The compositions described in Table 30 to Table 32 are artificial nail compositions for radical photocuring.

**(Table 30)**

| Artificial nail composition | | J-71 | J-72 | J-73 | J-74 | J-75 | J-76 | J-77 | J-78 | J-79 | J-80 |
|---|---|---|---|---|---|---|---|---|---|---|---|
| | A-38 | 40 | - | - | - | - | - | - | - | - | - |
| | A-51 | - | 40 | - | - | - | - | - | - | - | - |
| | A-54 | - | - | 40 | - | - | - | - | - | - | - |
| | A-57 | - | - | - | 40 | - | - | - | - | - | - |
| Polymer | A-95 | - | - | - | - | 40 | - | - | - | - | - |
| | A-96 | - | - | - | - | - | 40 | - | - | - | - |
| | A-97 | - | - | - | - | - | - | 40 | - | - | - |
| | A-98 | - | - | - | - | - | - | - | 40 | - | - |
| | A-99 | - | - | - | - | - | - | - | - | 40 | - |
| | A-100 | - | - | - | - | - | - | - | - | - | 40 |
| Polymerizable compound | B-105 | 25 | 25 | 25 | 25 | 25 | 25 | 25 | 25 | 25 | 25 |
| | B-116 | 12 | 12 | 12 | 12 | 12 | 12 | 12 | 12 | 12 | 12 |
| | B-111 | 5 | 5 | 5 | 5 | 5 | 5 | 5 | 5 | 5 | 5 |
| | B-117 | 5 | 5 | 5 | 5 | 5 | 5 | 5 | 5 | 5 | 5 |
| | B-114 | 7 | 7 | 7 | 7 | 7 | 7 | 7 | 7 | 7 | 7 |
| Polymerization initiator | B-201 | 3 | 3 | 3 | 3 | 3 | 3 | 3 | 3 | 3 | 3 |
| | B-203 | 3 | 3 | 3 | 3 | 3 | 3 | 3 | 3 | 3 | 3 |

**(Table 31)**

| Artificial nail composition | | J-81 | J-82 | J-83 | J-84 | J-85 | J-86 | J-87 | J-88 | J-89 | J-90 |
|---|---|---|---|---|---|---|---|---|---|---|---|
| | A-101 | 40 | - | - | - | - | - | - | - | - | - |
| | A-102 | - | 40 | - | - | - | - | - | - | - | - |
| | A-103 | - | - | 40 | - | - | - | - | - | - | - |
| | A-104 | - | - | - | 40 | - | - | - | - | - | - |
| Polymer | A-105 | - | - | - | - | 40 | - | - | - | - | - |
| | A-106 | - | - | - | - | - | 40 | - | - | - | - |
| | A-107 | - | - | - | - | - | - | 40 | - | - | - |
| | A-108 | - | - | - | - | - | - | - | 40 | - | - |
| | A-109 | - | - | - | - | - | - | - | - | 40 | - |
| | A-110 | - | - | - | - | - | - | - | - | - | 40 |
| Polymerizable compound | B-105 | 25 | 25 | 25 | 25 | 25 | 25 | 25 | 25 | 25 | 25 |
| | B-116 | 12 | 12 | 12 | 12 | 12 | 12 | 12 | 12 | 12 | 12 |
| | B-111 | 5 | 5 | 5 | 5 | 5 | 5 | 5 | 5 | 5 | 5 |
| | B-117 | 5 | 5 | 5 | 5 | 5 | 5 | 5 | 5 | 5 | 5 |
| | B-114 | 7 | 7 | 7 | 7 | 7 | 7 | 7 | 7 | 7 | 7 |
| Polymerzation initiator | B-201 | 3 | 3 | 3 | 3 | 3 | 3 | 3 | 3 | 3 | 3 |
| | B-203 | 3 | 3 | 3 | 3 | 3 | 3 | 3 | 3 | 3 | 3 |

**(Table 32)**

| Artificial nail composition | | J-91 | J-92 | J-93 | J-94 | H-11 | H-12 | H-13 | H-14 |
|---|---|---|---|---|---|---|---|---|---|
| | A-111 | 40 | - | - | - | - | - | - | - |
| | A-112 | - | 40 | - | - | - | - | - | - |
| | A-113 | - | - | 40 | - | - | - | - | - |
| Polymer | A-114 | - | - | - | 40 | - | - | - | - |
| | HA-1 | - | - | - | - | 40 | - | - | 40 |
| | HA-2 | - | - | - | - | - | 40 | - | - |
| | HA-3 | - | - | - | - | - | - | 40 | - |
| Polymerizable compound | B-105 | 25 | 25 | 25 | 25 | - | - | - | - |
| | B-116 | 12 | 12 | 12 | 12 | - | - | - | - |
| | B-111 | 5 | 5 | 5 | 5 | - | - | - | - |
| | B-117 | 5 | 5 | 5 | 5 | - | - | - | - |
| | B-114 | 7 | 7 | 7 | 7 | - | - | - | - |
| | B-104 | - | - | - | - | 27 | 27 | 54 | 54 |
| | B-102 | - | - | - | - | 27 | 27 | - | - |
| | B-201 | 3 | 3 | 3 | 3 | 3 | 3 | 3 | 3 |
| Polymerization initiator | B-203 | 3 | 3 | 3 | 3 | 3 | 3 | 3 | 3 |

The abbreviations in Table 30 to Table 32 other than those described above are as follows.

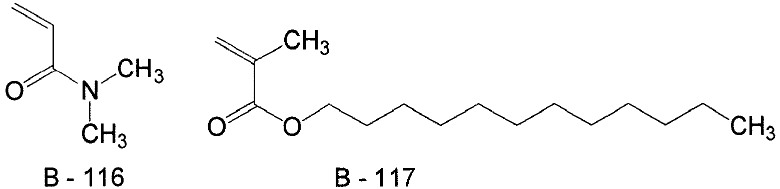

The B-116 and B-117 used were both manufactured by Tokyo Chemical Industry Co., Ltd.

### Evaluation of artificial nail

### <Adhesion-2>

Adhesion of the artificial nail obtained was evaluated in accordance with JIS K 5600-5-6 Adhesion (cross-cut method). The test results were classified as follows in accordance with JIS K 5600-5-6 from judgment of the state of the surface of the cross-cut section where peeling occurred.

### -Evaluation criteria-

0: edge of cut was completely smooth, no peeling in any lattice cell.
1: small amount of peeling of coating observed at intersection of cuts, affected cross-cut section was definitely no greater than 5%.
2: peeling of coating occurred along the edge of cuts and/or intersections, and affected cross-cut section was definitely greater than 5% but no greater than 15%.
3: coating partially or completely peeled off along edges of cuts and/or various sections of lattice cells were partially or completely peeled, affected cross-cut section was definitely greater than 15% but no greater than 35%.
4: coating partially or completely peeled off along edges of cuts and/or several lattice cells were partially or completely peeled, affected cross-cut section was definitely no greater than 35%.
5: the degree of peeling was such that it could not be classified as level 4.

### <Water resistance-2>

The artificial nail obtained was placed in tap water (pH 6.4) at 55°C and stirred at 200 rpm for 5 hours. After stirring, the artificial nail was taken out, and the surface state thereof was visually examined and evaluated using the evaluation criteria below.
A: peel-off did not occur.
B: peel-off did not occur, but slight lifting was observed at extremity.
C: slight peel-off occurred.
D: peel-off occurred.

### (Examples 93 to 129 and Comparative Examples 23 to 30)

### <Evaluation of artificial nail formed by radical curing and having upper layer provided>

The artificial nail produced in Formation Example 2 was evaluated in terms of removability-2, adhesion-2, and water resistance-2, and an aged artificial nail was subjected to the same evaluation. The results are shown in Table 33 and Table 34. Aging of the artificial nail was carried out by leaving it stored under an environment of 25°C to 35°C and 30% to 50%RH.

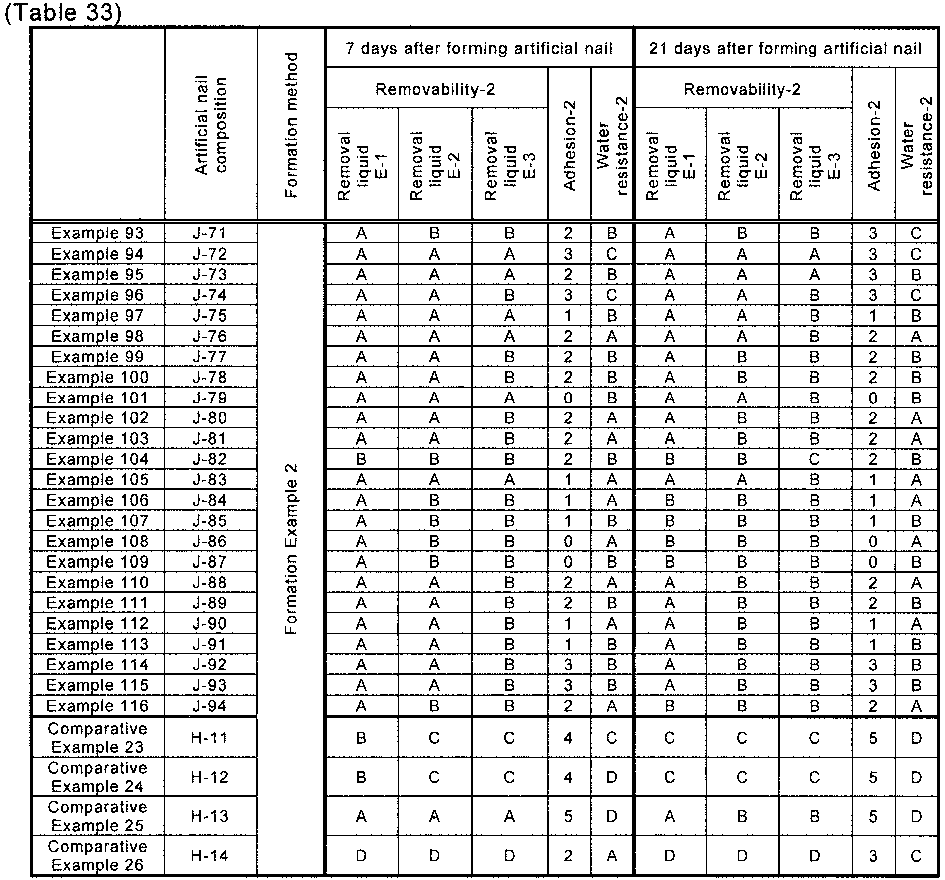

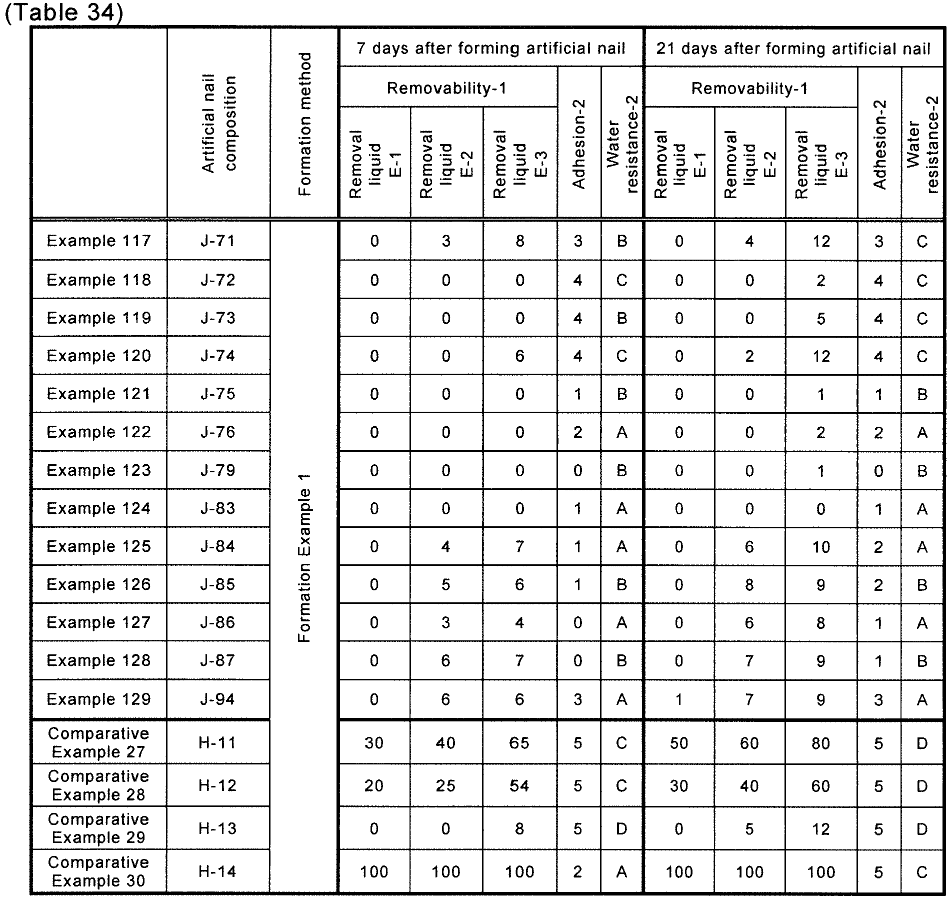

It can be seen from the results of Table 33 and Table 34 that the artificial nail obtained with the artificial nail composition of the present invention can be easily removed using an acid aqueous solution even when it is cured by exposure to light, and high adhesion can be maintained. It can also be seen that the artificial nail of the present invention has little degradation of performance even when time has elapsed after its formation.

## Claims

1. An artificial nail composition comprising
(Component A) an amino group-containing polymer.

2. The artificial nail composition according to Claim 1, wherein Component A is a polymer having an amino group in a side chain.

3. The artificial nail composition according to Claim 1 or 2, wherein the amino group is a secondary amino group and/or a tertiary amino group.

4. The artificial nail composition according to any one of Claims 1 to 3, wherein the amino group is a tertiary amino group.

5. The artificial nail composition according to any one of Claims 1 to 4, wherein Component A is a polymer comprising a structure represented by Formula (I) below in a side chain, 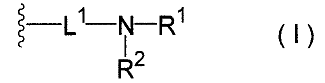 wherein in Formula (I) R¹ and R² independently denote a hydrogen atom or an alkyl group having 1 to 10 carbons, at least two selected from the group consisting of R¹, R², and L¹ may be bonded to each other to form a ring, L¹ denotes an alkylene group having 1 to 20 carbons, and the wavy line portion denotes the position of bonding to another structure.

6. The artificial nail composition according to Claim 5, wherein R¹ and R² are independently alkyl groups having 1 to 4 carbons.

7. The artificial nail composition according to any one of Claims 1 to 6, wherein Component A is a urethane-based polymer.

8. The artificial nail composition according to Claim 7, wherein Component A is a urethane-based polymer having a polycarbonate structure or a polyester structure.

9. The artificial nail composition according to Claim 8, wherein Component A is a urethane-based polymer having a polycarbonate structure.

10. The artificial nail composition according to any one of Claims 1 to 6, wherein Component A is a polymer comprising a monomer unit represented by Formula (II) below, 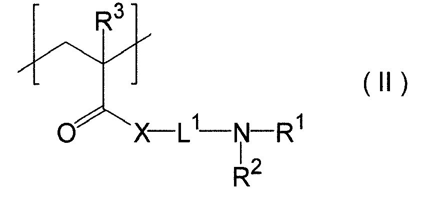 wherein in Formula (II) R¹ and R² independently denote a hydrogen atom or an alkyl group having 1 to 10 carbons, at least two selected from the group consisting of R¹, R², and L¹ may be bonded to each other to form a ring, L¹ denotes an alkylene group having 1 to 20 carbons, R³ denotes a hydrogen atom or an alkyl group having 1 to 3 carbons, and X denotes an oxygen atom or an NH group.

11. The artificial nail composition according to Claim 10, wherein Component A is a copolymer comprising a monomer unit represented by Formula (II) above and a monomer unit derived from a compound selected from the group consisting of a styrene compound and a (meth)acrylate compound other than a monomer unit represented by Formula (II) above.

12. The artificial nail composition according to any one of Claims 1 to 9, wherein Component A is a polymer comprising a monomer unit represented by Formula (III) below, 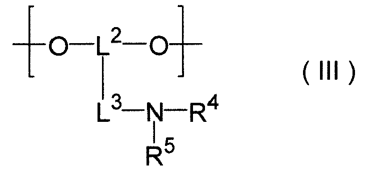 wherein in Formula (III) R⁴ and R⁵ independently denote a hydrogen atom or an alkyl group having 1 to 10 carbons, at least two selected from the group consisting of R⁴, R⁵, and L³ may be bonded to each other to form a ring, L² denotes a trivalent linking group, and L³ denotes an alkylene group having 1 to 20 carbons.

13. The artificial nail composition according to any one of Claims 1 to 12, wherein the artificial nail composition further comprises (Component B) a polymerizable compound and (Component C) a photopolymerization initiator.

14. An artificial nail comprising a layer formed by drying and/or exposing to light the artificial nail composition according to any one of Claims 1 to 13.

15. A method for forming an artificial nail, comprising
a step of forming a coated film by coating a human or animal nail or another artificial nail with the artificial nail composition according to any one of Claims 1 to 13, and
a step of forming an artificial nail by drying and/or exposing to light the coated film.

16. A nail art kit comprising
the artificial nail composition according to any one of Claims 1 to 13 and
a removal liquid.
